(19) Europäisches Patentamt
European Patent Office
Office européen des brevets

(11) Numéro de publication: **0 236 215 B1**

# (12) FASCICULE DE BREVET EUROPEEN

(45) Date de publication du fascicule du brevet: **10.01.90**

(21) Numéro de dépôt: **87400376.7**

(22) Date de dépôt: **20.02.87**

(51) Int. Cl.[4]: **C07C 69/76**, C07C 69/82, C07C 69/75, C07C 69/757, C07C 69/753, C07C 43/23, C09K 19/20, C09K 19/30, C09K 19/32, C09K 19/12

**(54) Difluoro-2,2' Alcoxy-4 Hydroxy-4' biphenyles et leurs dérivés, leur procédé de fabrication et leur utilisation dans des dispositifs d'affichage à cristeaux liquides.**

(30) Priorité: **28.02.86 FR 8602858**

(43) Date de publication de la demande:
**09.09.87 Bulletin 87/37**

(45) Mention de la délivrance du brevet:
**10.01.90 Bulletin 90/2**

(84) Etats contractants désignés:
**CH DE FR GB IT LI NL**

(56) Documents cités:
**EP-A- 0 153 826**
**GB-A- 2 071 649**
**GB-A- 2 098 987**

**CHEMICAL ABSTRACTS, vol. 101, 1984, page 681, résumé no. 130382r, Columbus, Ohio, US; & JP-A-59 76 029 (CHISSO CORP.) 28-04-1984**

(73) Titulaire: **COMMISSARIAT A L'ENERGIE ATOMIQUE, 31/33, rue de la Fédération, F-75015 Paris(FR)**

(72) Inventeur: **Vauchier, Claude, Villard-Bozon route de Theys, F-38570 Goncelin(FR)**
Inventeur: **Vinet, Françoise, 52, rue Thiers, F-38000 Grenoble(FR)**

(74) Mandataire: **Mongrédien, André et al, c/o BREVATOME 25, rue de Ponthieu, F-75008 Paris(FR)**

**EP 0 236 215 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet la synthèse de nouveaux cristaux liquides, utilisables en particulier dans des dispositifs d'affichage à cristaux liquides mettant en jeu l'effet de biréfringence contrôlé électriquement (BCE).

L'effet BCE correspond à une déformation sous champ électrique d'une phase nématique à anisotropie diélectrique$\Delta\varepsilon$ négative, $\Delta\varepsilon$ représentant la différence entre la constante diélectrique $\varepsilon_a$ parallèle au grand axe moléculaire du cristal et la constante diélectrique $\varepsilon_b$ perpendiculaire à ce grand axe.

Dans les dispositifs à cristaux liquides, il est nécessaire d'utiliser des matériaux présentant un taux élevé de multiplexage, c'est-à-dire donnant la possibilité d'adresser électriquement un nombre important de lignes de l'écran afin de visualiser un haut taux d'informations. Ce taux de multiplexage k peut s'exprimer en fonction de la tension appliquée aux bornes du dispositif (V) et de la tension de seuil de déformation du cristal ($V_S$) par la formule :

$$k < \left( \frac{V^2 + V_S^2}{V^2 - V_S^2} \right)^2$$

Pour un taux de multiplexage donné, il est nécessaire d'avoir un angle de basculement moléculaire ($\phi_M$) au centre de la cellule à cristaux liquides le plus grand possible pour obtenir le meilleur contraste entre les deux états noir et blanc du cristal sous l'action du champ électrique. Pour de faibles angles de basculement $\phi_M$ est directement lié à la tension de seuil de déformation du cristal $V_S$ et au rapport des constantes élastiques de flexion $K_{33}$ et d'éventail $K_{11}$ du cristal liquide. En effet, $\phi_M$ est donné par la formule :

$$\Phi_M = \frac{V^2 - V_S^2}{V^2 (\frac{2}{3} + \left|\frac{\Delta\epsilon}{\epsilon_a}\right| - \eta_{31} V_S^2}$$

$$\text{avec } V_S = 2\pi\sqrt{\frac{\pi K_{33}}{\Delta\epsilon}}$$

$$\Delta\epsilon = \epsilon a - \epsilon b$$

$$\eta_{31} = \frac{1 - K_{33}}{K_{11}}$$

Dans ces conditions, la biréfringence induite électriquement est alors de la forme :

$$\Delta n = \left( \frac{\sin^2 \Phi}{n_o^2} + \frac{\cos^2 \Phi}{n_e^2} \right)^{1/2} - n_0$$

$$\text{avec } \Phi \simeq \Phi_M \cos\frac{\pi}{e} Z$$

dans laquelle $n_e$ et $n_o$ sont les indices extraordinaire et ordinaire du cristal, e l'épaisseur du cristal liquide et Z sa position dans la cellule.

L'intensité relative transmise par le dispositif à cristal liquide entre deux polariseurs croisés est donnée par la relation :

$$\frac{I}{I_0} = \sin^2 \pi \frac{\Delta n\ e}{\lambda}$$

dans laquelle $\Delta n$ est égal à $n_e$-$n_o$, et $\lambda$ est la longueur d'onde du faisceau lumineux éclairant le dispositif. Il en résulte que les paramètres les plus importants du matériau sont $K_{33}/K_{11}$, $\Delta n$,$\Delta\varepsilon$ ainsi que la gamme de mésomorphisme $\Delta T$ du cristal.

Parmi ces paramètres, le rapport $K_{33}/K_{11}$ joue un rôle important, car il détermine la raideur de la pente de la courbe de transfert électro-optique et donc la multiplexabilité du matériau utilisé ; aussi, ce rapport doit être le plus grand possible.

Selon les composés nématiques utilisés, ce rapport peut varier de 0,5 à 3, la valeur la plus courante étant située autour de 1. Pour avoir une valeur plus élevée du rapport $K_{33}/K_{11}$, on peut envisager d'augmenter la valeur de $K_{33}$ ou de diminuer la valeur de $K_{11}$.

Une valeur élevée de $K_{33}$ n'est pas intéressante dans les dispositifs d'affichage, car elle implique l'utilisation de tensions de commande élevées. En effet, la tension de seuil de déformation du cristal $V_s$ est proportionnelle à la valeur de la constante élastique de déformation en flexion $K_{33}$ suivant la formule :

$$V_s = 2\pi \sqrt{\frac{\pi K_{33}}{|\Delta\varepsilon|}}$$

Il est donc préférable de diminuer la valeur de $K_{11}$, ce qui correspond à diminuer les interactions intermoléculaires entre les molécules du cristal liquide. Généralement, les molécules de cristal liquide nématique sont formées d'une partie centrale rigide comportant des groupements phényle et d'une ou deux parties flexibles situées aux extrémités de la partie rigide.

Plusieurs solutions peuvent être envisagées pour diminuer les interactions entre des molécules de ce type. L'une de ces solutions est de remplacer un cycle phényle de la partie rigide par un cyclohexane ou par un noyau bicyclo-octane. En effet ce dernier noyau par sa forme pratiquement globulaire et par ses liaisons complètement saturées ne favorise pas les interactions intermoléculaires et ceci permet de minimiser la valeur de $K_{11}$ et d'augmenter le rapport $K_{33}/K_{11}$. Une seconde solution est de substituer un ou plusieurs atomes d'hydrogène du noyau phényle par un élément électro-négatif tel que le fluor.

Récemment, on a synthétisé de nouveaux cristaux liquides pour lesquels le rapport $K_{33}/K_{11}$ a une valeur élevée. Ces nouveaux cristaux liquides sont par exemple :

- les alkyl bicyclo (2.2.2.) octane carboxylate d'alkyl fluorophényle comme le composé BC055F répondant à la formule :

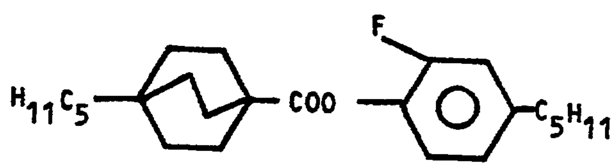

- les 4-(trans-4-n alkylcyclohexyl)-4'-alkyl-2,2'difluoro biphényle comme le composé BCH52FF de formule :

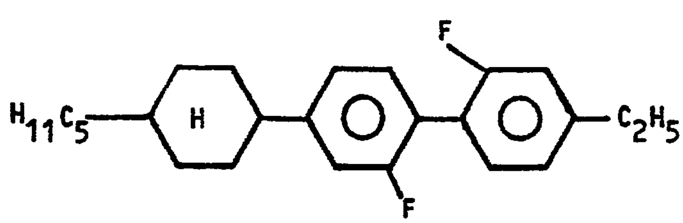

- et des bicyclohexylcarbonitriledialkyle ou alkylalkylène.

De tels composés possèdent les caractéristiques suivantes :

| Composé | $K_{33}/K_{11}$ | Δn | Δε |
|---|---|---|---|
| | 1. | 0,13 | –1 |
| $BCO_{55}F$ | 1,35 | 0,06 | –0,75 |
| $BCH_{52}FF$ | 1,7 | 0,99 | –0,47 |

Ainsi, comme on peut le voir sur ce tableau où l'on a donné à première ligne les caractéristiques requises pour les dispositifs d'affichage mettant en jeu l'effet BCE, ces composés sont seulement performants sur le plan des constantes élastiques, la valeur du rapport $K_{33}/K_{11}$ étant élevée. En revanche, leurs autres caractéristiques ( n et ) ne permettent pas d'obtenir les propriétés électro-optiques requises pour les dispositifs d'affichage. De ce fait, on a envisagé d'utiliser ces composés en mélange avec d'autres cristaux liquides pour améliorer ces propriétés électro-optiques, mais ceci a pour conséquence de diminuer de façon importante la valeur du rapport $K_{33}/K_{11}$.

La présente invention a précisément pour objet de nouveaux composés utilisables comme cristaux liquides, qui présentent un ensemble de paramètres électro-optiques satisfaisant mieux les conditions requises pour une utilisation dans des dispositifs d'affichage à cristaux liquides mettant en jeu l'effet BCE.

Ces composés qui sont des dérivés de difluoro-2,2′ alcoxy-4 hydroxy-4′ biphényle répondent à la formule :

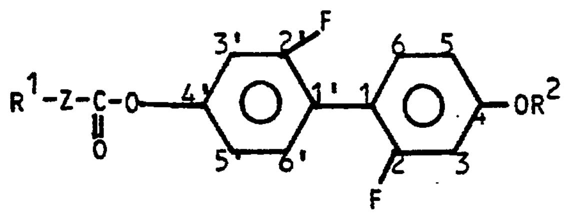

(I)

dans laquelle $R^1$ représente un radical alkyle ou alcoxy linéaire ou ramifié de 1 à 12 atomes de carbone, Z représente une simple liaison ou un radical choisi parmi :

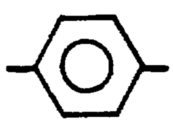 ; 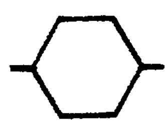 ; 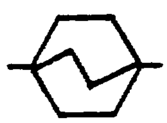 ou 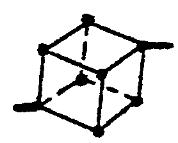

à condition que $R_1$ représente un radical alkyle lorsque Z repésente une simple liaison, et $R^2$ représente un radical alkyle linéaire ou ramifié de 1 à 12 atomes de carbone.

Les composés décrits ci-dessus ont une structure qui permet d'obtenir des valeurs élevées du rapport $K_{33}/K_{11}$. En effet, ces molécules sont longues et rigides. De ce fait, il est difficile d'obtenir des déformations en flexion sous l'action d'un champ électrique. Ainsi, la valeur de $K_{33}$ est élevée. Par ailleurs, la présence d'atomes de fluor en position 2 et 2′ sur le biphényle provoque une torsion des deux noyaux phényle l'un par rapport à l'autre. Les deux positions les plus favorables correspondent à des angles de torsion égaux à 30° et 120°. La molécule n'est donc plus plane et l'empilement compact des molécules les unes par rapport aux autres n'est pas aussi favorisé que dans le cas de molécules planes. De ce fait, les interactions intermoléculaires sont affaiblies et la valeur de la constante d'éventail $K_{11}$ du composé diminue. Cet effet s'accentue lorsqu'on passe de Z représentant une simple liaison à Z représentant un radical et il augmente dans le sens suivant : phénylène, cyclohexylène, bicyclooctylène ou radical dérivé du cubane.

A titre d'exemples des composés de formule (I) de l'invention, on peut citer les composés suivants :
- les dérivés de difluoro-2,2′ alcoxy-4 hydroxy-4′ biphényle de formule :

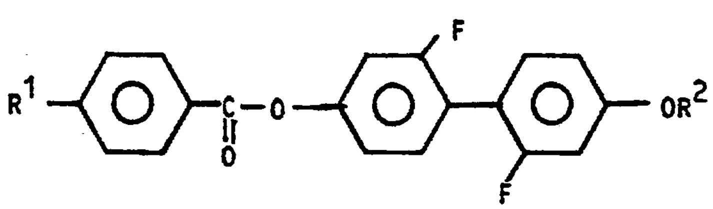

(X)

dans laquelle $R^1$ est le radical butyloxy et $R^2$ est le radical éthyle ou dans laquelle $R^1$ est le radical décycloxy et $R^3$ est le radical (S) méthyl-2 butyle ;
- les dérivés de difluoro-2,2′ alcoxy-4 hydroxy-4′ biphényle de formule :

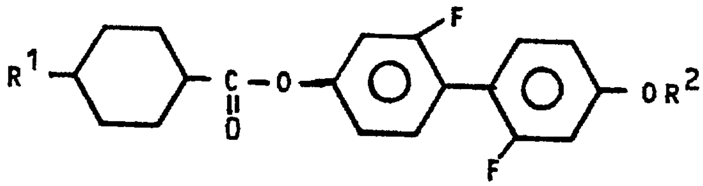

(XI)

dans laquelle $R^1$ est un radical alkyle de 3 à 5 atomes de carbone et dans laquelle $R^2$ est un radical alkyle de 2 à 5 atomes de carbone;
- les dérivés de difluoro-2,2′ alcoxy-4 hydroxy-4′ de fomule :

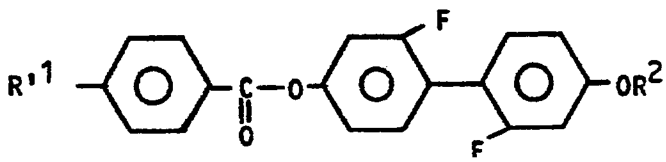

(XII)

dans laquelle $R^1$ est un radical alkyle de 6 à 8 atomes de carbone et $R^2$ est un radical alkyle de 2 à 5 atomes de carbone ; et
- le dérivé de difluoro-2,2′ alcoxy-4 hydroxy-4′ biphényle de formule :

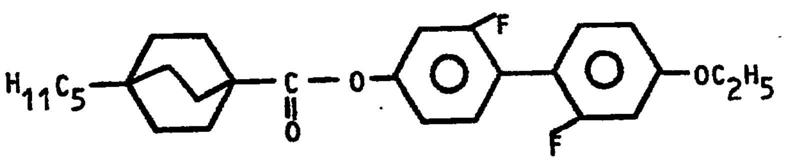

(XIII)

Les dérivés de difluoro-2,2′ alcoxy-4 hydroxy-4′ biphényle décrits ci-dessus ont également des propriétés intéressantes en ce qui concerne l'intervalle de mésomorphisme $\Delta T$ du cristal liquide et les paramètres électro-optiques $\Delta n$ et $\Delta \varepsilon$.

Aussi, ces composés peuvent être utilisés pour la réalisation des écrans de dispositifs d'affichage à cristaux liquides utilisant l'effet de biréfringence contrôlé électriquement. Dans ce cas, on utilise au moins un composé de l'invention pour former l'écran. On peut ainsi utiliser un seul composé ou un mélange de plusieurs composés répondant à la formule (I) de l'invention. L'utilisation d'un mélange permet en particulier d'obtenir des caractéristiques appropriées, par exemple d'élargir l'intervalle de mésomorphisme vers les températures inférieures à la température ambiante.

On peut aussi utiliser un ou plusieurs composés de formule (I) de l'invention en association avec d'autres cristaux liquides pour former une composition de cristal liquide présentant le meilleur compromis entre tous les paramètres tels que le rapport des constantes élastiques, la gamme de mésomorphisme, etc.

Aussi, l'invention a également pour objet un mélange de cristaux liquides, comportant :
- au moins un composé de formule (I), et
- au moins un composé choisi parmi :

a) les bases de Schiff répondant à la formule :

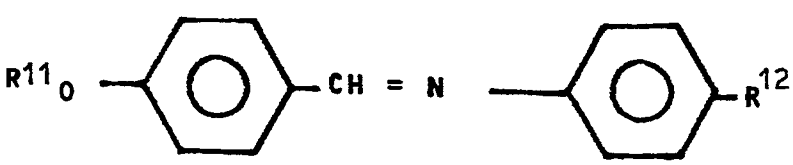

(XIV)

dans laquelle $R^1$ et $R^2$ qui peuvent être identiques ou différents, sont des radicaux alkyle de 1 à 7 atomes de carbone ;

b) les 1-(alkylcyclohexyl)-2-(alkylfluorobiphénylyl) éthane de formule :

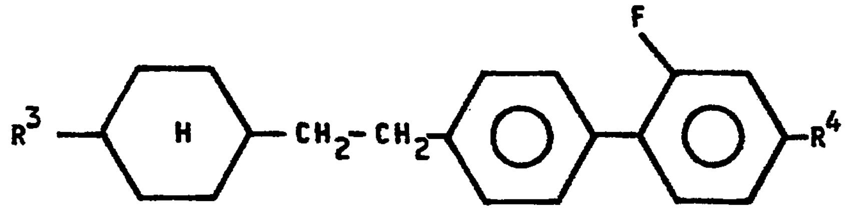

(XV)

dans laquelle $R^3$ et $R^4$ qui peuvent être identiques ou différents, sont des radicaux alkyle de 1 à 7 atomes de carbone, et

c) les akylbicylcooctane carboxylates d'alkyl fluorophényle de formule :

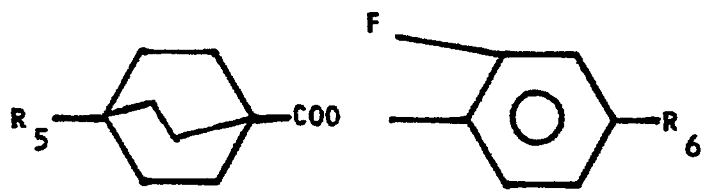

(XVI)

dans laquelle $R_5$ et $R_6$ qui peuvent être identiques ou différents, sont des radicaux alkyle de 1 à 7 atomes de carbone,

d) les cyclohexylbiphényle de formule :

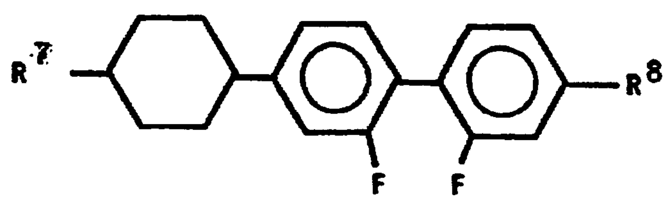

(XVII)

dans laquelle $R_7$ est un radical alkyle de 1 à 12 atomes de carbone et $R_8$ est un radical alkyle ou alkoxy de 1 à 12 atomes de carbone.

Dans ces mélanges, la teneur minimale en composé(s) de formule (I) est de préférence d'au moins 10% en poids, et généralement d'au moins 20% en poids.

Grâce à l'association d'au moins un composé de formule (I) avec un ou plusieurs composés choisis parmi les composés a), b), c) et d) mentionnés ci-dessus, on obtient une composition de cristal liquide présentant le meilleur compromis entre tous les paramètres : rapport de constantes élastiques $K_{33}/K_{11} \geq 1,3$, large gamme de mésomorphisme, plus faible viscosité, meilleure orientation homéotrope dans les cellules.

Les différents constituants du mélange sont pour la plupart des produits connus, et ils peuvent être préparés par des procédés classiques.

Ainsi, les 1-(alkylcyclohexyl)-2-(alkylfluorobiphénylyl) éthanes de formule (XV) peuvent être préparés en utilisant la méthode décrite dans le brevet anglais GB-A 2 133 795.

A titre d'exemples de composés de ce type susceptibles d'être utilisés dans l'invention, on peut citer le 1-(trans-4-n-éthylcyclohexyl)-2-[2'-fluoro-4'-(2-éthyl)-4-biphénylyl] éthane (I22) de formule :

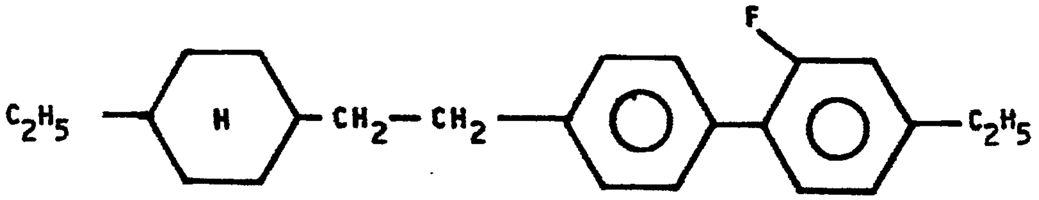

()

Le 1-(trans-4-n-propylcyclohexyl)-2- [2'fluoro-4'-(2-pentyl)-4-biphénylyl] éthane (I35) de formule :

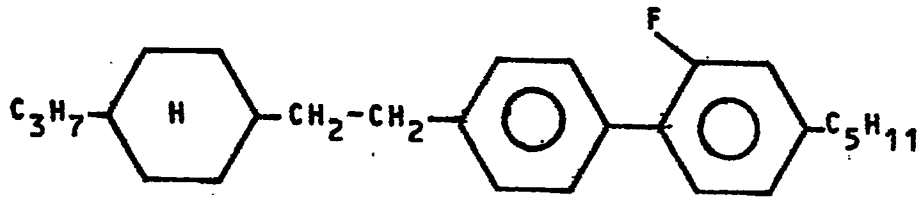

(IXX)

Le 1-(trans-4-n-propylcyclohexyl)-2- [2'-fluoro-4'-(2-éthyl) -4-biphénylyl] éthane (I32) de formule :

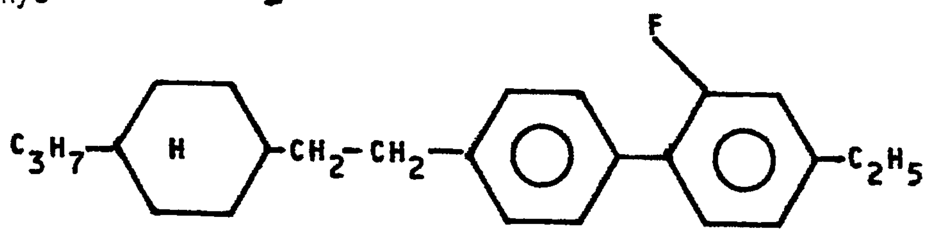

(XX)

Le 1-(trans-4-n-pentylcyclohexyl)-2- [2'-fluoro-4'-(2-éthyl)- 4-biphénylyl] éthane (I52) de formule :

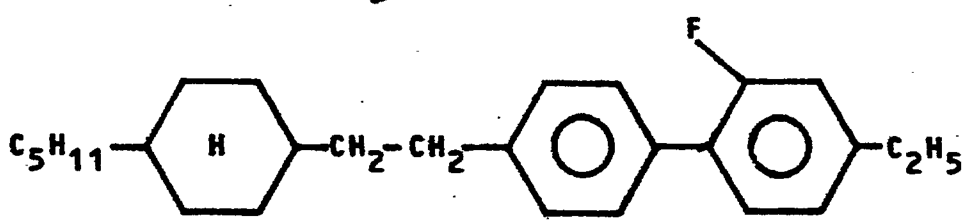

(XXI)

Les alkylbicylcooctane carboxylates d'alkylfluorophényle de formule (XVI) peuvent être préparés par la méthode décrite par G.W.Gray et S.M.Kelly dans Mol. Cryst. Liq. Cryst. (1981), vol.75, p.109-119.

A titre d'exemples de composés de ce type susceptibles d'être utilisés, on peut citer le 4-n-pentyl-bicyclooctane carboxylate de 2-fluoro-4-n-pentyl-phényle (BCO55F) de formule :

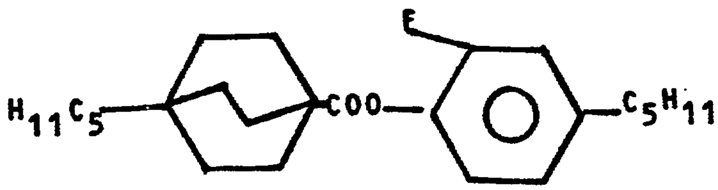

(XXII)

et le 4-n-pentyl-bicyclooctane carboxylate de 2-fluoro-4-n-heptylphényle (BCO57F) de formule :

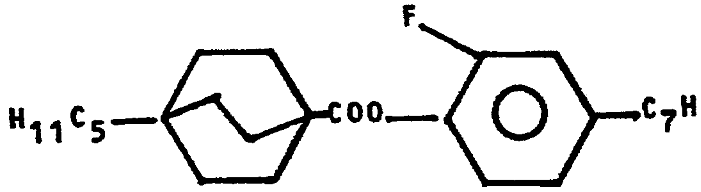 (XXIII)

Les cyclohexylbiphényles de formule (XVII) peuvent être préparés par les techniques décrites dans le brevet américain US-A-4 415 470. A titre d'exemple de tels composés, on peut citer le cyclohexylbiphényle de formule :

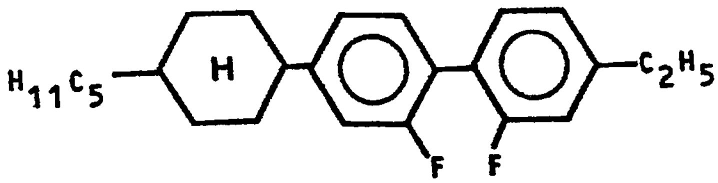 (XXIV)

que l'on désignera ci-après par les termes BCH52FF.

Les bases de Schiff peuvent être préparées en utilisant la méthode décrite par H. Kelker et B. Scheuble dans Angew. Chem. Internat. Edit. vol.8 (1969) n°11, p.884-885. A titre d'exemple de bases de Schiff susceptibles d'être utilisées dans l'invention, on peut citer enfin le p-méthoxy-benzylidène de p- butylaniline (MBBA) de formule :

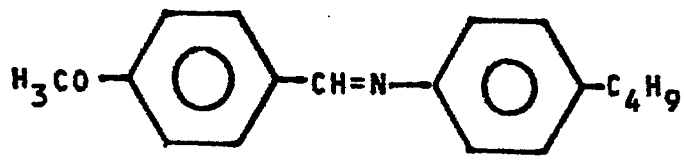 (XXV)

Les cristaux liquides mentionnés ci-dessus ont les propriétés données dans le tableau 1 en annexe, et on peut améliorer certaines de ces propriétés en leur ajoutant un ou plusieurs composés de formule (I) de l'invention.

La présente invention a également pour objet un procédé de préparation des composés répondant à la formule (I) ci-dessus.

Ce procédé consiste à faire réagir un chlorure d'acide de formule $R^1$-Z-COCL dans laquelle $R^1$ et Z ont la signification donnée ci-dessus avec un composé répondant à la formule :

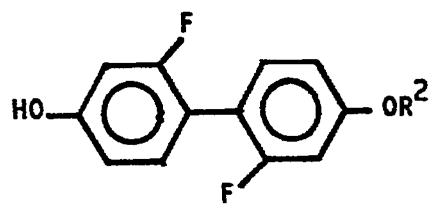 (II)

dans laquelle $R^2$ est un radical alkyle linéaire ou ramifié ayant de 1 à 12 atomes de carbone.

Cette réaction peut être effectuée dans un solvant anhydre tel que la pyridine, à la température ambiante.

Le chlorure d'acide de formule $R^1$-Z-COCL peut être préparé à partir de l'acide carboxylique correspondant de formule $R^1$-Z-COOH par des procédés classiques, par exemple par réaction de l'acide avec le chlorure de thionyle.

Lorsque Z représente une simple liaison ou un radical phénylène de formule

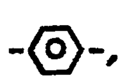

les acides carboxyliques correspondants sont généralement disponibles commercialement ou peuvent être préparés par des procédés classiques.

Lorsque Z représente le radical bicyclo (2.2.2) octylène de formule

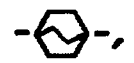

et $R^1$ représente un radical alkyle, on peut synthétiser les acides correspondants en utilisant la méthode décrite par G.W.Gray et S.M. Kelly pour la préparation de bicyclo (2.2.2) octanes disubstitués en 1.4 (J. Chem. Soc. Perkin II (1981), p.26-31 et brevet anglais GB-A-2069483). Selon cette méthode, on part des dérivés bromés corres-pondants de formule

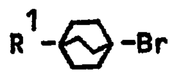

qui peuvent être obtenus à partir de $R^1$-$CH_2$-$COCH_3$ comme il est décrit dans les publications mentionnées ci-dessus.

Lorsque Z représente le radical bicyclo (2.2.2) octylène et $R^1$ représente un radical alcoxy, on peut synthétiser l'acide correspondant en utilisant la méthode décrite par W.Adcock et A.N. Abeywickrema dans J. Org. Chem. 47, p.2951-2957 (1982) pour la préparation de l'acide

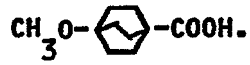

Lorsque Z représente le radical cyclohexylène de formule

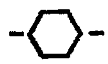

et $R^1$ représente un radical alkyle ou alcoxy, on peut synthétiser l'acide correspondant en utilisant la méthode décrite dans le brevet japonais 80/53244 du 18/4/80 (Chemical Abstract vol. 93, 1980, no 167 736b).

Lorsque Z représente le radical dérivé du cubane de formula:

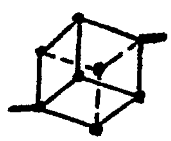

on peut synthétiser l'acide correspondant en utilisant les méthodes décrites par Gray et al. dans Mol. Cryst. Liq. Cryst., 1983, vol.98, pp.425-431.

L'invention a également pour objet les difluoro-2,2', alcoxy-4 hydroxy-4' biphényles de formule (II) qui forment la structure de base des cristaux liquides nématiques de l'invention.

Ces composés de formule (II) peuvent être synthétisés à partir du nitro-3 amino-4 anisole par un procédé correspondant au schéma réactionnel suivant :

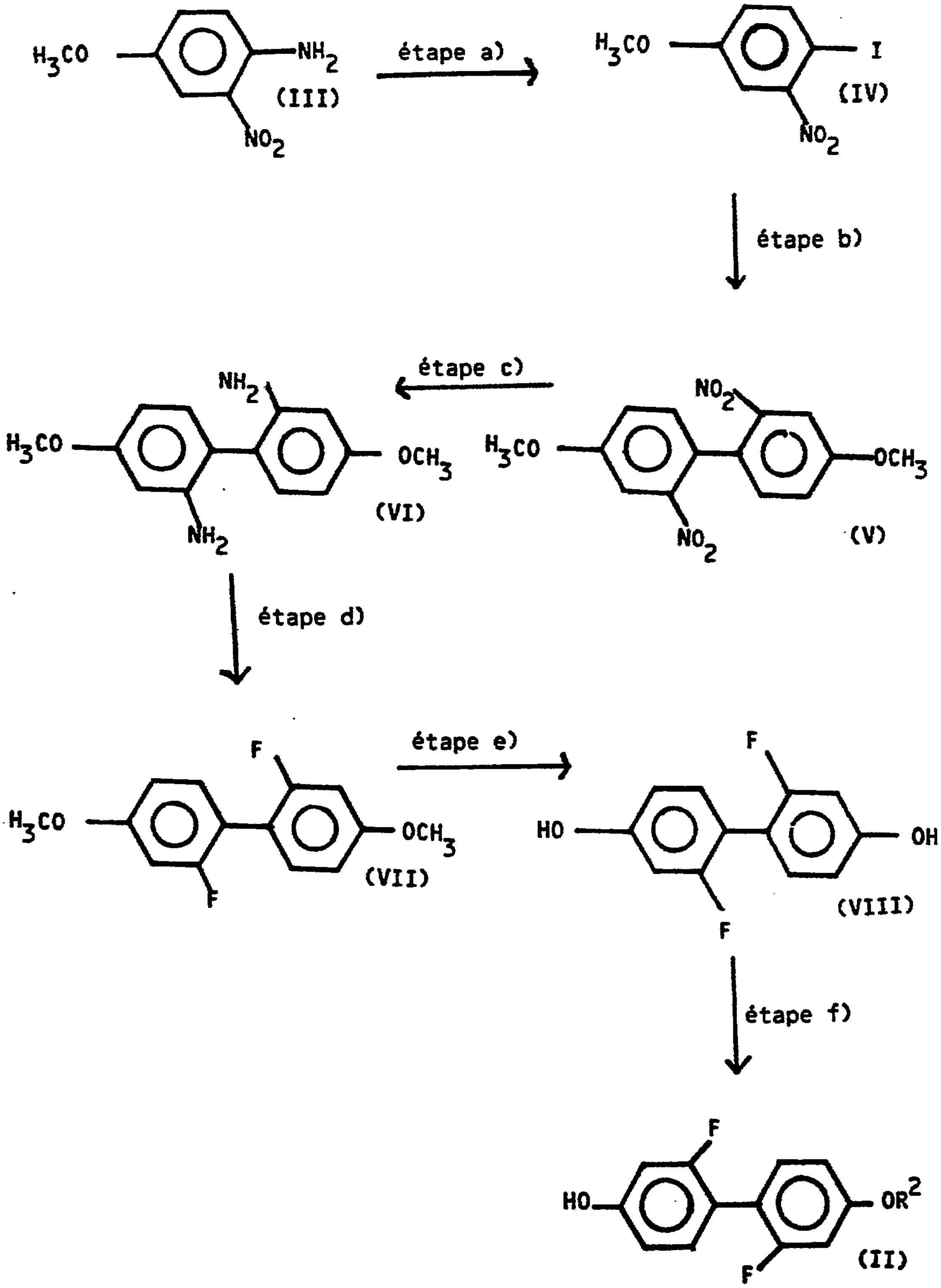


(III) (IV) (V) (VI) (VII) (VIII) (II)

Ainsi, le procédé de préparation des difluoro-2,2′ alcoxy-4 hydroxy-4′ biphényles de formule (II) comprend les étapes suivantes :

a) convertir le nitro-3 amino-4 anisole de formule (III) en nitro-3 iodo-4 anisole de formule (IV) par réaction avec de l'acide nitreux ou une solution aqueuse de nitrite de métal alcalin et un iodure de métal alcalin,

b) condenser deux molécules du nitro-3 iodo-4 anisole de formule (IV) pour obtenir le dinitro-2,2′ diméthoxy-4,4′ biphényle de formule (V),

c) réduire les groupements $NO_2$ du dinitro-2,2′ diméthoxy-4,4′ biphényle de formule (V) en groupements $NH_2$ pour obtenir le diamino-2-2′ diméthoxy-4,4′ biphényle de formule (VI),

d) remplacer les groupements $NH_2$ du diamino-2,2′ diméthoxy-4,4′ biphényle de formule (VI) par des atomes de fluor pour obtenir le difluoro-2,2′ diméthoxy-4,4′ biphényle de formule (VII),

e) remplacer les groupes méthoxy du difluoro-2,2′ diméthoxy-4,4′ biphényle de formule (VII) par des groupes hydroxyle pour obtenir le difluoro-2,2′ dihydroxy-4,4′ biphényle de formule (VIII), et

f) faire réagir le difluoro-2,2′ dihydroxy-4,4′ biphényle de formule (VIII) avec un dérivé halogéné de formule $R^2X$ dans laquelle X représente un halogène et $R^2$ représente un radical alkyle de 1 à 12 atomes de carbone, pour obtenir le difluoro-2,2′ alcoxy-4 hydroxy-4′ biphényle de formule (II).

La première étape a) qui consiste à remplacer le substituant $NH_2$ par un atome d'iode peut être effectuée selon le processus décrit par H. Heaney et I.T. Millar dans Organic Syntheses, vol.40, p.105, soit en faisant réagir le dérivé aminé avec de l'acide nitreux ou une solution aqueuse de nitrite de métal alcalin et un iodure de métal alcalin. Cette réaction peut être effectuée dans de l'acide chlorhydrique concentré en ajoutant goutte à goutte à la solution une solution de nitrite de sodium dans l'eau, puis une solution d'iodure de sodium.

La deuxième étape b) qui consiste à condenser deux molécules de nitro-3 iodo-4 anisole pour former le dérivé dinitro-2,2′ diméthoxy-4,4′ biphényle, peut être effectuée en utilisant la réaction de condensation de Ullmann décrite dans Organic Syntheses, vol.3, p.339 et dans J. Chem. Soc. p.2658 (1958). Cette réaction peut être effectuée dans un solvant organique tel que le nitrobenzène en utilisant du cuivre en poudre et en chauffant à une température de 200 à 220°C.

La troisième étape c) qui consiste à réduire les groupements $NO_2$ du composé de formule (V) en groupement $NH_2$ peut etre réalisée par des méthodes classiques, par exemple par action d'acide chlorhydrique en présence de catalyseurs à base de fer, de zinc et d'étain, comme le chlorure d'étain II dihydraté.

La quatrième étape d) qui consiste à remplacer les groupements $NH_2$ par des atomes de fluor peut être effectuée en utilisant la réaction de Schiemann-Balz décrite par exemple dans Organic Reactions, vol.5, p.193-228 (1949). Selon cette réaction, on obtient tout d'abord un fluoborate de diazonium et l'on décompose ensuite ce sel pour obtenir le dérivé fluoré correspondant en libérant de l'azote et du trifluorure de bore. Cette réaction peut être effectuée dans l'acide chlorhydrique concentré par action d'acide nitreux ou d'une solution aqueuse de nitrite de métal alcalin et d'une solution de tétrafluoroborate de métal alcalin. Après précipitation du fluoborate de diazonium, on sépare celui-ci de la solution, on le sèche puis on le chauffe lentement à une température suffisante pour le décomposer et récupérer ainsi le dérivé difluoré.

La cinquième étape e) qui consiste à remplacer les groupes méthoxy par des groupes hydroxyle peut être effectuée en utilisant la réaction décrite par N. Carr G.W. Gray et S.M. Kelly dans Mol. Cryst. Liq. Cryst., 1985, vol.129, pp.301-313, pour convertir le dérivé méthoxyphényl bicyclo (2.2.2) octane en hydroxyphényl bicyclo (2.2.2) octane. Cette réaction peut être effectuée en utilisant de l'acide bromhydrique et de l'acide acétique en milieu aqueux comme il est décrit dans cet article.

La sixième f) qui consiste à transformer l'un des groupements hydroxyle en groupement alcoxy par réaction du composé de formule (VIII) avec un dérivé halogéné de formule $R^2X$ dans laquelle X représente un halogène tel que le brome et $R^2$ a la signification donnée précédemment, peut être effectuée. En fin de réaction, on sépare par chromatographie le monoéther du diéther.

Les composés de formule II ainsi préparés sont très utiles pour la synthèse d'autres composés car leur structure constitue une base pour la réalisation de nombreux cristaux liquides.

D'autres caractéristiques et avantages de l'invention apparaîtront mieux à la lecture des exemples suivant donnés bien entendu à titre illustratif et non limitatif.

La figure unique annexée représente en coupe verticale un dispositif d'affichage à cristal liquide utilisant les composés de l'invention.

Exemple 1 : préparation du difluoro-2,2′ éthoxy-4 hydroxy-4′ biphényle de formule :

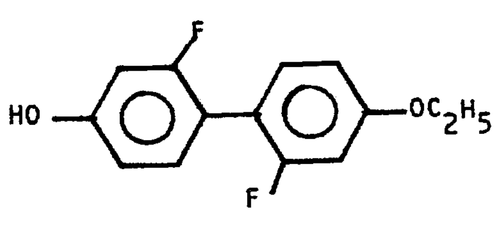

(IX)

a) préparation du nitro-3 iodo-4 anisole de formule (IV)

On met en suspension 72,3g de nitro-3 amino-4 anisole dans 210ml d'acide chlorhydrique concentré et on refroidit la suspension à une température de 0 à 5°C, puis on lui ajoute 140g de glace. On ajoute ensuite goutte à goutte une solution de 32,2g de nitrite de sodium dans 133ml d'eau. On agite le mélange pendant 1/4h à 5°C puis on le filtre et on verse le filtrat sur une solution de 294g d'iodure de potassium dans 800ml d'eau. On filtre le précipité obtenu, on le lave successivement à l'eau, avec une solution de sulfite de sodium à 5% dans l'eau et encore à l'eau et on le sèche sous vide. On purifie le produit brut par distillation sous vide, on obtient ainsi 90g du composé de formule (IV), ce qui correspond à un rendement de 75%. La température d'ébullition du composé est de 173°C sous 0,9mm de Hg et sa température de fusion est de 61°C.

b) préparation du dinitro-2,2' diméthoxy-4,4' biphényle de formule (V)

On chauffe à 190°C une solution de 50g du nitro-3 iodo-4 anisole obtenu en a) dans 50ml de nitrobenzène et on lui ajoute, sur une durée d'1/4h, 45g de cuivre en poudre. On agite fortement le mélange et on le chauffe 1/2h de plus à 200-210°C. On laisse refroidir le mélange et on ajoute du toluène. On filtre la solution, puis on distille les solvants, c'est-à-dire le toluène et le nitrobenzène. On obtient ainsi un produit solide brun que l'on purifie par recristallisations successives dans le méthanol, ce qui donne 16,5g d'aiguilles jaunes et brillantes du composé de formule (V). Sa température de fusion est de 132°C. Le rendement est de 60%.

c) préparation du diamino-2,2' diméthoxy-4,4' biphényle de formule (VI)

On met en suspension dans 130ml d'éthanol 10g du dinitro-2,2' diméthoxy-4,4' biphényle obtenu précédemment avec 74,2g de chlorure d'étain II dihydraté. On agite fortement la solution et on lui ajoute goutte à goutte 20ml d'acide chlorhydrique concentré en réglant la vitesse d'addition de l'acide chlorhydrique de manière à ne pas avoir d'échauffement trop violent du milieu réactionnel. Après 20 min, on obtient une solution limpide et homogène. On agite encore 2h de plus, puis on refroidit la solution avec de la glace et on verse lentement une solution de soude concentrée à 2 $mol.l^{-1}$. On précipite ainsi du tétrachlorure d'étain IV ainsi que le diamino-2,2' diméthoxy-4,4' biphényle. On rajoute alors de la soude pour ajuster le pH de la solution à une valeur nettement basique de l'ordre de 14. Ainsi, on fait disparaître le précipité minéral et on peut filtrer le diamino-2,2' diméthoxy-4,4' biphényle. On lave le précipité plusieurs fois à l'eau, puis on le reprend à l'éther éthylique, on le filtre et on le sèche sur sulfate de sodium. On purifie ensuite le produit obtenu par recristallisations dans l'éthanol. On obtient ainsi 6,85g du composé de formule (VI). Le point de fusion est de 108-109°C et le rendement est de 85%.

d) préparation du difluoro-2,2' diméthoxy-4,4' biphényle de formule (VII)

On met en suspension 6,8g du composé obtenu à l'étape c) dans 8ml d'acide chlorhydrique concentré. On ajoute alors 8ml de dioxanne, puis on ajoute goutte à goutte une solution de 4,5g de nitrite de sodium dans 12ml d'eau à une température de 0°C. Immédiatement après et toujours à 0°C, on ajoute goutte à goutte une solution de 13,4g de tétrafluoborate de sodium dans 30ml d'eau. Une demi-heure après la fin de l'addition du réactif fluoré, on filtre le précipité formé, on le lave avec de l'eau glacée, puis on le sèche sous vide à la température ambiante. On met en suspension la poudre séchée dans 200ml de xylène anhydre, puis on porte lentement le mélange au reflux. Vers 110°C, le dégagement de fumée blanche de $BF_3$ commence et on arrête la réaction lorsque le dégagement gazeux cesse. On filtre le mélange, puis on élimine le xylène par distillation sous pression atmosphérique. On dissout alors le résidu obtenu à chaud dans de l'éthanol et on le recristallise, ce qui donne 3g du difluoro-2,2' diméthoxy-4,4' biphényle de formule (VII) ayant un point de fusion de 70°C. Le rendement est de 43%.

e) préparation du difluoro-2,2' dihydroxy-4,4' biphényle de formule (VIII)

On dissout les 3g du composé obtenu à l'étape d) dans 25ml d'une solution aqueuse d'acide bromhydrique à 48-50% et 83ml d'une solution d'acide bromhydrique dans l'acide acétique à 45%, et on met au reflux pendant 20h. On ajoute ensuite la solution refroidie à 100ml d'eau, puis on l'agite avec de l'éther éthylique. On lave la phase organique avec une solution de carbonate de sodium à 10%, puis avec de l'eau, et on la sèche sur sulfate de sodium. On recristallise le produit obtenu dans du chloroforme, et on obtient ainsi 1,7g de difluoro-2,2' dihydroxy-4,4' biphényle de formule (VIII) ayant un point de fusion supérieur à 210°C (sublimation). Le rendement est de 64%.

f) préparation du difluoro-2,2' éthoxy-4 hydroxy-4' biphényle de formule (IX)

On dissout 1g du composé de formule (VIII) obtenu dans l'étape e) dans 250ml de méthyléthyl cétone anhydre. On ajoute 4g de carbonate de potassium et on agite vigoureusement. On ajoute alors goutte à

goutte une solution de 0,5g de bromméthane dans 10ml de méthyléthyl cétone. On chauffe au reflux le mélange pendant 20h, puis on verse la solution sur 400ml d'eau glacée et on extrait avec du chloroforme. On lave la phase organique à l'eau et on la sèche sur sulfate de sodium. On chromatographie le résidu obtenu sur colonne de silice en éluant avec un mélange chloroforme-éther diéthylique (95/5) en volume). Ainsi, on récupère d'abord le difluoro-2,2' diéthoxy-4,4' biphényle, puis 0,45g du difluoro-2,2' éthoxy-4 hydroxy-4' biphényle de formule (IX) qui a un point de fusion de 106,5°C. Le rendement est de 40%.

Exemple 2

Préparation d'autres composés répondant à la formule (II).

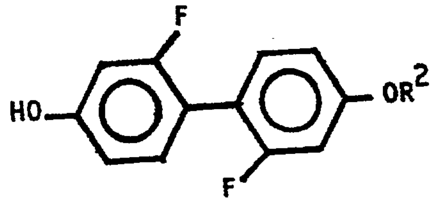

(II)

On suit le même mode opératoire que dans l'exemple 1 pour préparer les composés du tableau 2 joint. La température de fusion de ces composés est donnée également dans le tableau 2.

Dans le cas du composé de formule (II) comportant le radical 2-(méthylbutyloxy)-4 hydroxy-4' biphényle de formule :

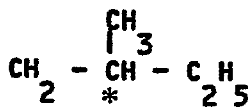

on suit également le mode opératoire de l'exemple 1 mais dans l'étape f), on utilise comme réactif le (S) bromo-1 méthyl-2 butane qui a été préalablement synthétisé à partir du (S) méthyl-2 butanol-1 par la méthode décrite par Gray G.W. et Mc Donnell D.G. (Mol. Cryst. Liq. Cryst., 1976, vol.37, 189-211).

Exemple 3

Préparation du difluoro-2,2' éthoxy-4 para-n-butoxy benzoate-4' biphényle de formule :

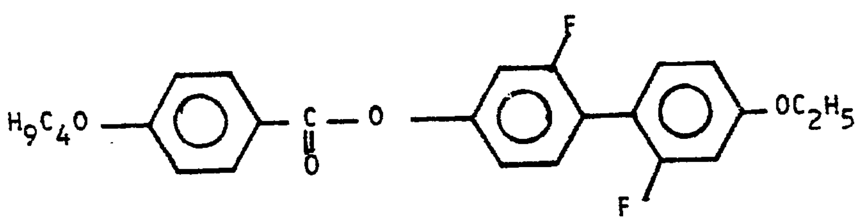

$(X)_1$

désigné ci-après par PF402.

1) Préparation du chlorure d'acide para-n-butoxy benzoïque

On porte au reflux pendant 2h une solution de 5g d'acide para-n-butoxy benzoïque dans 8ml de chlorure de thionyle. On distille alors le chlorure de thionyle, puis on distille sous pression réduite et sous courant d'azote sec le chlorure d'acide attendu. On obtient ainsi le chlorure d'acide para-n-butoxy benzoïque de formule :

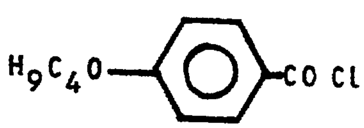

2) Préparation du difluoro-2,2' éthoxy-4 para-n-butoxy benzoate-4' biphényle de formule (X)

On dissout 0,4g du difluoro-2,2' éthoxy-4 hydroxy-4' biphényle obtenu dans l'exemple 1 dans 20ml de pyridine anhydre. On ajoute goutte à goutte à la solution fortement agitée 0,41g du chlorure d'acide obtenue précédemment. On agite le mélange à la température ambiante pendant 24h. Puis on le verse sur 50ml de solution glacée d'acide chlorhydrique à 10%. On filtre le précipité obtenu, on le lave avec une solution d'acide chlorhydrique à 10% pour éliminer toute la pyridine, puis à l'eau jusqu'à l'obtention d'eaux de lavages neutres. On reprend le produit par du chloroforme et on le sèche sur du sulfate de sodium. On chromatographie le produit brut sur une colonne de silice, on élue au chloroforme et on recristallise dans le méthanol. On obtient ainsi 0,54g de difluoro-2,2' éthoxy-4 para-n-butoxy benzoate-4' biphényle, ce qui correspond à un rendement de 80%.

Ce produit est mésogène et présente une large plage nématique comprise entre la température de passage de l'état solide à l'état nématique $T_{KN}$ et la température de passage de l'état nématique à l'état isotrope $T_{NI}$, $T_{KN}$ étant de 124°C et $T_{NI}$ de 193°C.

On prépare de la même façon le difluoro-2,2' ((S) méthyl-2 butyloxy)-4 para-n-décyloxy benzoate-4' biphényle.

Les caractéristiques des deux composés obtenus dans l'exemple 3 sont données dans le tableau 3 annexé.

Exemple 4

Préparation du difluoro-2,2' éthoxy-4 para-n-pentyl-cyclohexanoate trans-4' biphényle de formule :

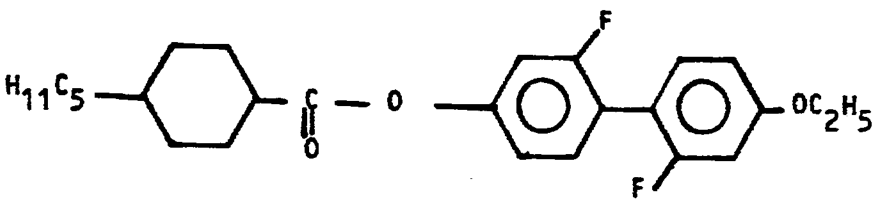
(XI)1

désignée ci-après par CHF52.

1) Préparation du chlorure d'acide para-n-pentyl cyclohexanoîque trans.

On porte au reflux une solution de 5g d'acide para-n-pentyl cyclohexanoïque trans dans 8ml de chlorure de thionyle pendant 2h. On élimine alors le chlorure de thionyle par distillation sous pression atmosphérique, puis on distille le chlorure d'acide sous pression réduite et sous courant d'azote sec. On obtient ainsi un produit ayant une température d'ébullition de 87°C sous 0,5 mm Hg.

2) Préparation du difluoro-2,2' éthoxy-4 para-n-pentyl-cyclohexanoate trans-4' biphényle de formule (XI).

On dissout 0,3g du difluoro-2,2' éthoxy-4 hydroxy-4' biphényle obtenu dans l'exemple 1 dans 15ml de pyridine anhydre. On ajoute ensuite goutte à goutte à cette solution 0,3g du chlorure d'acide obtenu précédemment. On agite le mélange pendant 24h à la température ambiante, puis on le verse sur 50ml d'une solution d'acide chlorhydrique à 10%. On filtre le précipité obtenu et on le lave avec de l'acide chlorhydrique à 10%, puis avec de l'eau. On reprend le produit par du chloroforme et on le sèche sur du sulfate de sodium. On chromatographie le composé brut obtenu sur une colonne de silice en réalisant l'élution au moyen de chloroforme, puis on le recristallise dansle méthanol. On obtient ainsi 0,4g de difluoro-2,2' éthoxy-4 para-n-pentyl cyclohexanoate-trans-4' biphényle, ce qui correspond à un rendement de 79%. Ce produit est mésogène et présente une large plage nématique car $T_{KN}$ est de 74°C et $T_{NI}$ de 170°C.

Exemple 5

On prépare comme dans l'exemple 4, les composés de formule :

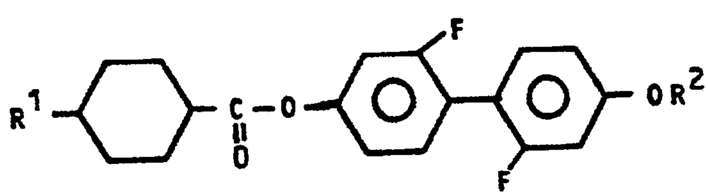
(XI)

du tableau 4 annexé.

Les caractéristiques des composés obtenus sont également données dans le tableau 4 annexé.

Exemple 6 :

Préparation du difluoro-2,2' éthoxy-4 para-n-hexyl benzoate-4' biphényle de formule :

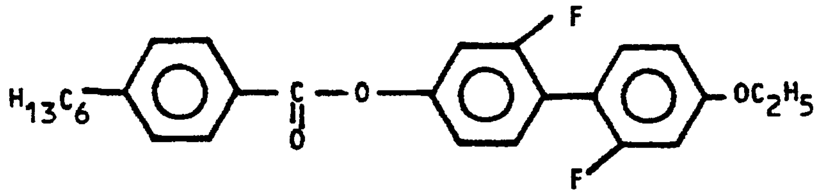

(XII)1

désignée ci-après par PF62.

1) Préparation du chlorure d'acide para-n-hexyl benzoïque

On porte au reflux une solution de 3g d'acide para-n-hexyl benzoïque dans 7ml de chlorure de thionyle pendant 2h. On élimine le chlorure de thionyle par distillation sous pression atmosphérique, puis on distille le chlorure d'acide sous pression réduite et sous courant d'azote sec. On obtient ainsi un produit ayant une température d'ébullition de 153°C sous 1,2 mm Hg.

2) Préparation du difluoro-2,2' éthoxy-4 para-n-hexyl benzoate-4' biphényle de formule (XII).

On dissout 0,3g du difluoro-2,2' éthoxy-4 hydroxy-4' biphényle obtenu dans l'exemple 1 dans 15ml de pyridine anhydre. On ajoute ensuite goutte à goutte à cette solution 0,32g du chlorure d'acide obtenu précédemment. On agite le mélange pendant 24h à la température ambiante, puis on le verse sur 50ml d'une solution d'acide chlorhydrique à 10%. On filtre le précipité obtenu et on le lave avec de l'acide chlorhydrique à 10%, puis avec de l'eau. On reprend le produit par du chloroforme et on le sèche sur du sulfate de sodium. On chromatographie le composé brut obtenu sur une colonne de silice en réalisant l'élution au moyen de chloroforme, puis on le recristallise dans le méthanol. On obtient ainsi 0,42g de difluoro-2,2' éthoxy-4 para-n-hexyl benzoate-4' biphényle, ce qui correspond à un rendement de 80%. Ce produit est mésogène et présente une large plage nématique car $T_{KN}$ est de 74,4°C et $T_{NI}$ de 142,5°C.

Exemple 7

On prépare comme dans l'exemple 6 les composés de formule :

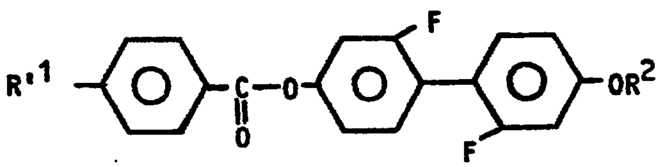

(XII)

mentionnés dans le tableau 5 annexé, en utilisant le même mode opératoire que dans l'exemple 6. Les caractéristiques des produits obtenus sont également données dans le tableau 5.

Exemple 8 :

Préparation du difluoro-2,2' éthoxy-4 (n-pentyl bicyclo (2.2.2) octanoate)-4' biphényle de formule :

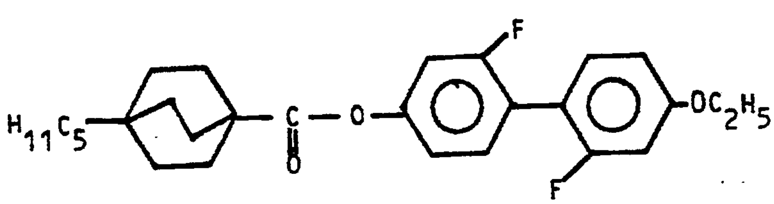

(XIII)

désigné ci-après par BCOF52.

1) Préparation du chlorure d'acide n-pentyl-1 bicyclo (2.2.2) octanoïque-4

L'acide n-pentyl-4 bicyclo (2.2.2) octanoïque est obtenu par les méthodes décrites dans la littérature et citées précédemment (p.8) (Température de fusion 160°C).

On porte au reflux une solution de 1g de cet acide dans 4ml de chlorure de thionyle pendant 2heures. On élimine le chlorure de thionyle par distillation sous pression atmosphérique. Le chlorure d'acide obtenu est utilisé tel quel, sans autres purifications.

2) Préparation du difluoro-2,2' éthoxy-4 (n-pentyl-4 bicyclo (2.2.2) octanoate-1)-4' biphényle deformule (XIII)

On dissout 0,3g du difluoro-2,2' éthoxy-4 hydroxy-4' biphényle obtenu dans l'exemple 1 dans 15ml de pyridine anhydre. On ajoute ensuite goutte à goutte à cette solution 0,35g du chlorure d'acide obtenue précédemment. On agite le mélange pendant 24h à la température ambiante, puis on le verse sur 50ml d'une solution d'acide chlorhydrique à 10%. On filtre le précipité obtenu et on le lave avec de l'acide chlorhydrique à 10%, puis avec de l'eau. On reprend le produit par du chloroforme et on le sèche sur du sulfate de sodium. On chromatographie le composé brut obtenu sur une colonne de silice en réalisant l'élution au moyen de chloroforme, puis on le recristallise dans le méthanol. On obtient 0,41g de difluoro-2,2' éthoxy-4 (n-pentyl-4 bicyclo(2.2.2) octanoate)-4' biphényle, ce qui correspond à un rendement de 76%. Ce produit est mésogène et présente une large plage nématique car $T_{KN}$ est de 102°C et $T_{NI}$ de 205,5°C.

Les dérivés de difluoro-2,2' alcoxy-4 hydroxy-4' biphényle répondant à la formule (I), en particulier ceux des exemples 2 à 5, peuvent être utilisés comme cristaux liquides dans le dispositif d'affichage représenté en coupe verticale sur la figure annexée.

Sur cette figure, on voit que le dispositif d'affichage comprend deux parois isolantes et transparentes 1 et 3 et des joints d'étanchéité 5 et 7 délimitant une cavité interne 8 remplie de cristal liquide. Les deux parois isolantes sont revêtues intérieurement d'un système d'électrodes à bandes croisées 9 et 11 permettant de déterminer les zones d'affichage. Un système de polariseurs croisés 13 et 15 est disposé de part et d'autre des parois 1 et 3 et un système de commande 17 permet d'appliquer une tension électrique sur les électrodes voulues. En fonctionnement, on applique à certaines des électrodes les tensions voulues pour déformer le cristal liquide dans les zones correspondant à ces électrodes et obtenir ainsi l'affichage.

Ce dispositif de commande des électrodes peut être du type de celui décrit dans le brevet européen EP-A-0055966 du Commissariat à l'Energie Atomique.

Dans ce dispositif, on peut utiliser comme cristal liquide les composés de formule (I) de l'invention, en particulier sous la forme de mélanges de ces composés ou de mélanges de ces composés avec d'autres cristaux liquides. Les exemples qui suivent illustrent la réalisation de tels mélanges.

Exemple 9

On prépare le mélange suivante :
- 21% en poids de PF65 du tableau 5
- 16,5% en poids de PF64 du tableau 5
- 22% en poids de CHF44 du tableau 4
- 19,5% en poids de CHF43 du tableau 4
- 21% en poids de CHF33 du tableau 4

On obtient ainsi un mélange de cristaux liquides présentant une phase mésomorphe nématique dans la gamme de température allant d'une température inférieure à -10°C jusqu'à 138,2°C.

On mesure l'anisotropie diélectrique $\Delta\varepsilon$, l'anisotropie optique $\Delta n$ et le rapport $K_{33}/K_{11}$ du mélange obtenu et l'on obtient les valeurs suivantes :
$\Delta n$ = 0,1744 à 633 nm et à 20°C
$\Delta\varepsilon$ = -2,7
$K_{33}/K_{11}$ = 1,76 à 20°C

Exemple 10

On prépare un mélange de cristaux liquides comprenant :
- 53,7% en poids de I22 de formule (XVIII)
- 18,7% en poids de CHF33 du tableau 4
- 13,00% en poids de CHF43 du tableau 4
- 14,6% en poids de CHF44 du tableau 4

On obtient ainsi un mélange de cristaux liquides présentant une phase mésomorphe nématique dans la gamme de température allant d'une température < à -10°C jusqu'à 102,5°C. On mesure l'anisotropie optique $\Delta n$, le rapport $K_{33}K/_{11}$ et l'anisotropie diélectrique et l'on obtient les valeurs suivantes :
$\Delta n$ = 0,152 à 20°C (633nm)
$\Delta\varepsilon$ = -1,0
$K_{33}/K_{11}$ = 1,62 à 20°C

Exemple 11

On prépare un mélange de cristaux liquides comprenant :
- 54,1% en poids de MBBA de formule (XXV)
- 26,1% en poids de CHF33 du tableau 4
- 19,8% en poids de CHF43 du tableau 4

Ce mélange présente les caractéristiques suivantes :
- gamme de mésomorphisme = -3°C à 93°C
- anisotropie optique $\Delta n = 0{,}18$
- anisotropie diélectrique $\Delta\varepsilon = -1{,}2$
- rapport constantes élastiques $K_{33}/K_{11} > 1{,}4$

Exemple 12

On prépare un mélange de cristaux liquides comprenant :
- 38,8% en poids de I22 de formule (XVIII)
- 26,9% en poids de MBBA de formule (XXV)
- 14,4% en poids de CHF33 du tableau 4
- 9,7% en poids de CHF43 du tableau 4
- 10,2% en poids de CHF44 du tableau 4.

Ce mélange présente les caractéristiques suivantes :
- gamme de mésomorphisme : -24°C à 87°C
- anisotropie optique $\Delta n = 0{,}16$
- anisotropie diélectrique$\Delta\varepsilon = -0{,}85$
- rapport de constantes élastiques $K_{33}/K_{11} > 1{,}4$.

Exemple 13

On prépare un mélange de cristaux liquides comprenant :
- 47,7% en poids de BCH52FF de formule (XXIV)
- 20,8% en poids de CHF33 du tableau 4
- 16,5% en poids de CHF44 du tableau 4
- 15,0% en poids de CHF43 du tableau 4

Le mélange présente les caractéristiques suivantes :
- gamme de mésomorphisme : <-10°C à 117°C
- anistropie optique : $\Delta n = 0{,}13$
- anistropie diélectrique : $\Delta\varepsilon = -1{,}3$
- rapport de constantes élastiques : $K_{33}/K_{11} > 1{,}4$.

Exemple 14

On prépare un mélange de cristaux liquides comprenant :
- 19,5% en poids de BC055F de formule (XXII)
- 45,4% en poids de I22 de formule (XVIII)
- 5,6% en poids de CHF52 du tableau 4
- 16,9% en poids de CHF33 du tableau 4
- 12,6% en poids de CHF44 du tableau 4.

Le mélange présente les caractéristiques suivantes :
- gamme de mésomorphisme : <-10°C à 95°C
- anisotropie optique : $\Delta n = 0{,}112$
- anisotropie diélectrique :$\Delta\varepsilon = -0{,}9$
- rapport de constantes élastiques : $K_{33}/K_{11} > 1{,}4$.

Tableau 1

| Cristal liquide | Intervalle de mésomorphisme nématique | anisotropie optique $\Delta n$ | anisotropie diélectrique $\Delta\varepsilon$ | $K_{33}/K_{11}$ |
|---|---|---|---|---|
| 1-(alkylcyclohexyl) 2-(alkylfluoro-biphényl) éthane I22 | –10 à 102°C | 0,149 | –0,05 | 1,2 |
| alkylbicyclooctane carboxylates d'alkyl fluorophényl $BCO_{55}F$ | 26,3 à 64°C | 0,06 | –0,72 | 1,35 |
| p-méthoxy-benzylidène p'-butylaniline MBBA | 18° à 42°C | 0,17 | –0,4 | 1,32 |
| cyclohexylbiphényle $BCH_{52}FF$ | 27° à 78°C | 0,099 | –0,47 | 1,76 |

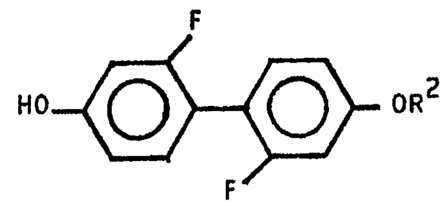

(II)

TABLEAU 2

| Composé de formule (II) | $R^2$ | Température de fusion Tf |
|---|---|---|
| difluoro-2,2' éthoxy-4 hydroxy-4' biphényle | $C_2H_5$ | 106,5°C |
| difluoro-2,2' n-propyloxy-4 hydroxy-4' biphényle | $C_3H_7$ | 93,7°C |
| difluoro-2,2' n-butyloxy-4 hydroxy-4' biphényle | $C_4H_9$ | 103,8°C |
| difluoro-2,2' n-pentyloxy-4 hydroxy-4' biphényle | $C_5H_{11}$ | 107°C |
| difluoro-2,2' (méthyl-2 butyloxy)-4 hydroxy-4' biphényle | $CH_2-\underset{*}{\overset{CH_3}{\overset{\vert}{C}H}}-C_2H_5$ | 113°C |

**TABLEAU 3**

Composé de formule :

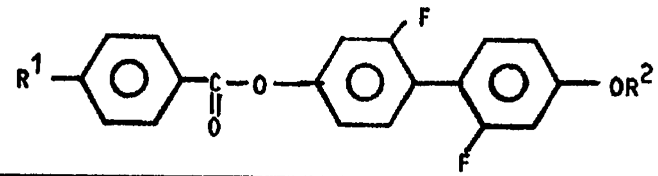

(X)

| Composé de formule (X) | Dénomination | $R^1$ | $R^2$ | $T_{KN}$ | $T_{NI}$ |
|---|---|---|---|---|---|
| difluoro-2,2' éthoxy-4 para-n-butoxy benzoate-4' biphényle | PF402 | $OC_4H_9$ | $C_2H_5$ | 124°C | 193°C |
| difluoro-2,2' ((S) méthyl-2 butyloxy)-4 para-n-décyloxy benzoate-4' biphényle | | $OC_{10}H_{21}$ | $CH_2-\overset{CH_3}{\underset{*}{C}H}-C_2H_5$ | 71°C | 109,9°C |

TABLEAU 4

Composé de formule : 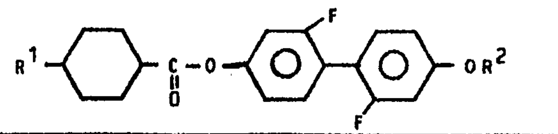 (XI)

| Composé de formule (XI) | Dénomination | $R^1$ | $R^2$ | $T_{KN}$ | $T_{NI}$ |
|---|---|---|---|---|---|
| difluoro-2,2' éthoxy-4 para-n-pentyl cyclohexanoate trans-4' biphényle | CHF52 | $C_5H_{11}$ | $C_2H_5$ | 74°C | 170°C |
| difluoro-2,2' n-propyloxy-4 para-n-pentyl cyclohexanoate trans-4' biphényle | CHF53 | $C_5H_{11}$ | $C_3H_7$ | 66°C | 156,7°C |
| difluoro-2,2' n-butyloxy-4 para-n-pentyl cyclohexanoate trans-4' biphényle | CHF54 | $C_5H_{11}$ | $C_4H_9$ | 65,4°C | 154,9°C |
| difluoro-2,2' n-propyloxy-4 para-n-butyl cyclohexanoate trans-4'biphényle | CHF43 | $C_4H_9$ | $C_3H_7$ | 61,6°C | 152,4°C |
| difluoro-2,2' n-butyloxy-4 para-n-butyl cyclohexanoate trans-4' biphényle | CHF44 | $C_4H_9$ | $C_4H_9$ | 51,3°C | 149,3°C |
| difluoro-2,2' n-propyloxy-4 para-n-propyl cyclohexanoate trans-4' biphényle | CHF33 | $C_3H_7$ | $C_3H_7$ | 56,7°C | 158,1°C |
| difluoro-2,2' n-butyloxy-4 para-n-butyl cyclohexanoate trans-4' biphényle | CHF34 | $C_3H_7$ | $C_4H_9$ | 41,1°C | 157,1°C |
| difluoro-2,2' ((S) méthyl) 2-butyloxy)-4 para-n-butyl cyclohexanoate trans-4' biphényle | CHF45* | $C_4H_9$ | $CH_2-\overset{CH_3}{\underset{*}{CH}}-C_2H_5$ | 35,8°C | 99,8°C |

TABLEAU 5

Composé de formule : 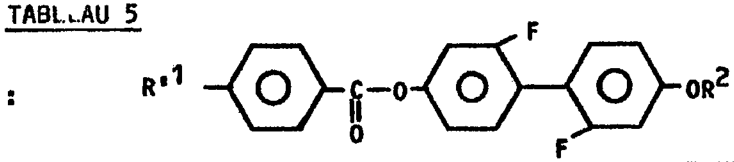 (XII)

| Composé de formule (XII) | Dénomination | $R'^1$ | $R^2$ | $T_{KN}$ | $T_{NI}$ |
|---|---|---|---|---|---|
| Difluoro-2,2' éthoxy-4 para-n-hexyl benzoate-4' biphényle | PF62 | $C_6H_{13}$ | $C_2H_5$ | 74,4°C | 142,5°C |
| Difluoro-2,2' n-propyloxy-4 para-n -hexyl benzoate-4' biphényle | PF63 | $C_6H_{13}$ | $C_3H_7$ | 74°C | 127°C |
| difluoro-2,2' n-butyloxy-4 para-n-hexyl benzoate-4' biphényle | PF64 | $C_6H_{13}$ | $C_4H_9$ | 55°C | 131°C |
| difluoro-2,2' n-pentyloxy-4 para-n-hexyl benzoate-4' biphényle | PF65 | $C_6H_{13}$ | $C_5H_{11}$ | 55°C | 123°C |
| difluoro-2,2' ((S) méthyl-2 butyloxy)-4 para-n-octyl benzoate-4' biphényle | PF85* | $C_8H_{17}$ | $CH_2-\underset{*}{C}H(CH_3)-C_2H_5$ | 54,8°C | 81,3°C |

## Revendications

1. Dérivé de difluoro-2,2′ alcoxy-4 hydroxy-4′ biphényle répondant à la formule :

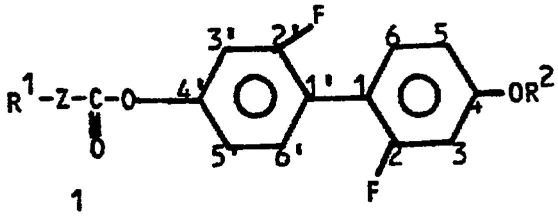

(I)

dans laquelle $R^1$ représente un radical alkyle ou alcoxy linéaire ou ramifié de 1 à 12 atomes de carbone, Z représente une simple liaison ou un radical choisi parmi :

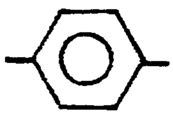 ; 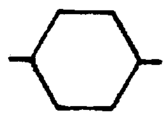 ; 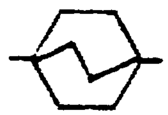 ou 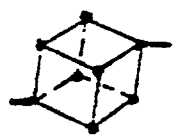

à condition que $R^1$ représente un radical alkyle lorsque Z représente une simple liaison, et $R^2$ représente un radical alkyle linéaire ou ramifié de 1 à 12 atomes de carbone.

2. Dérivé de difluoro-2,2′ alcoxy-4 hydroxy-4′ biphényle selon la revendication 1, caractérisé en ce qu'il répond à la formule :

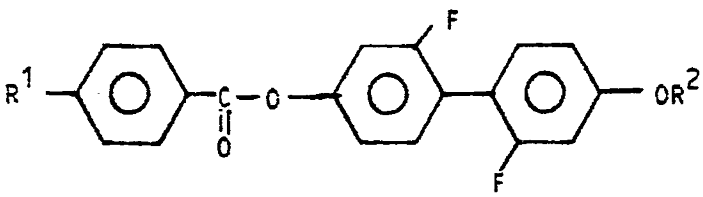

(X)

dans laquelle $R^1$ est le radical butyloxy et $R^2$ est le radical éthyle ou dans laquelle $R^1$ est le radical décyloxy et $R^2$ est le radical (S) méthyl-2 butyle.

3. Dérivé de difluoro-2,2′ alcoxy-4 hydroxy-4′ biphényle selon la revendication 1, caractérisé en ce qu'il répond à la formule :

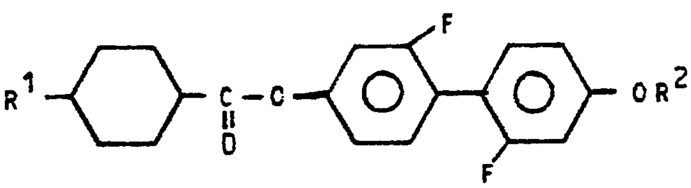

(XI)

dans laquelle $R^1$ est un radical alkyle de 3 à 5 atomes de carbone et $R^2$ est un radical alkyle de 2 à 5 atomes de carbone.

4. Dérivé de difluoro-2,2′ alcoxy-4 hydroxy-4′ biphényle selon la revendication 1, caractérisé en ce qu'il répond à la formule :

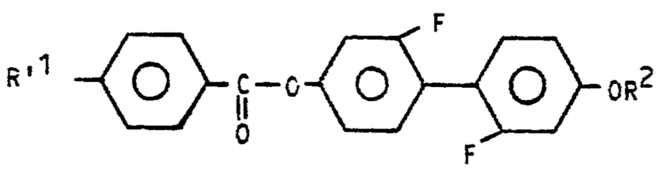

(XII)

dans laquelle $R^1$ est un radical alkyle de 6 à 8 atomes de carbone et $R^2$ est radical alkyle de 2 à 5 atomes de carbone.

5. Dérivé de difluoro-2,2′ alcoxy-4 hydroxy-4′ biphényle selon la revendication 1, caractérisé en ce qu'il répond à la formule :

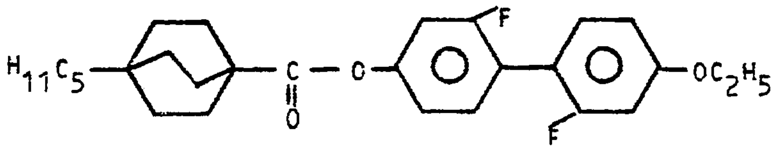 (XIII)

6. Procédé de préparation d'un dérivé de difluoro-2,2′ alcoxy-4 hydroxy-4′ biphényle répondant à la formule :

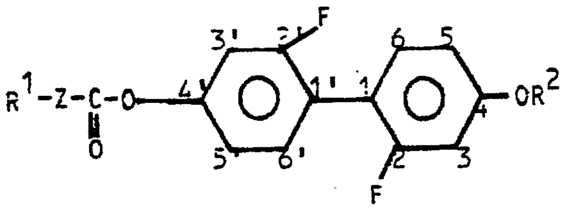 (I)

dans laquelle $R^1$ représente un radical alkyle ou alcoxy linéaire ou remifié ayant de 1 à 12 atomes de carbone, Z représente une simple liaison ou un radical choisi parmi :

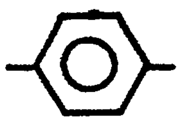 ; 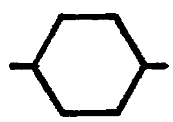 ; 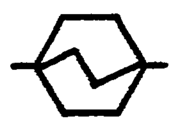 ou 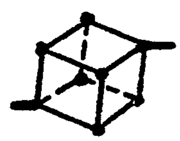

à condition que $R^1$ représente un radical alkyle lorsque Z représente une simple liaison, et $R^2$ représente un radical alkyle linéaire ou ramifié de 1 à 12 atomes de carbone, caractérisé en ce que l'on fait réagir un chlorure d'acide de formule $R^1$-Z-COCl dans laquelle $R^1$ et Z ont la signification donnée ci-dessus avec un composé de formule :

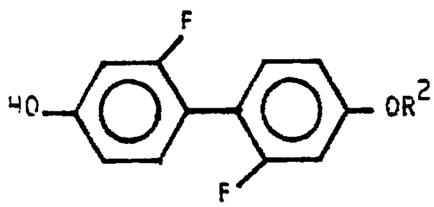 (II)

dans laquelle $R^2$ a la signification donnée ci-dessus.

7. Difluoro-2,2′ alcoxy-4 hydroxy-4′ biphényle répondant à la formule :

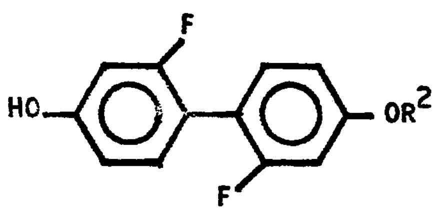 (II)

dans laquelle $R^2$ représente un radical alkyle linéaire ou ramifié de 1 à 12 atomes de carbone.

8. Difluoro-2,2′ alcoxy-4 hydroxy-4′ biphényle selon la revendication 7, caractérisé en ce que $R^2$ est choisi parmi les radicaux éthyle, propyle, butyle, n-pentyle et méthyl-2 butyle.

9. Procédé de préparation d'un difluoro-2,2′ alcoxy-4 hydroxy-4′ biphényle répondant à la formule :

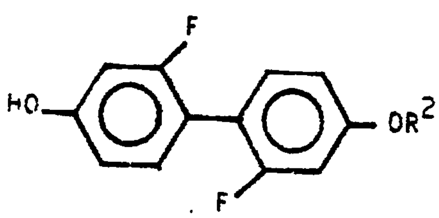 (II)

dans laquelle $R^2$ représente un radical alkyle linéaire ou ramifié de 1 à 12 atomes de carbone, caractérisé en ce qu'il comprend les étapes suivantes :

a) convertir le nitro-3 amino-4 anisole de formule :

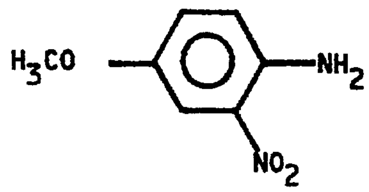 (III)

en nitro-3 iodo-4 anisole de formule :

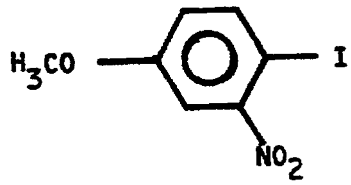 (IV)

par réaction avec de l'acide nitreux ou une solution aqueuse de nitrite de métal alcalin et un iodure de métal alcalin,
b) condenser deux molécules du nitro-3 iodo-4 anisole de formule (IV) pour obtenir le dinitro-2,2' diméthoxy-4,4' biphényle de formule :

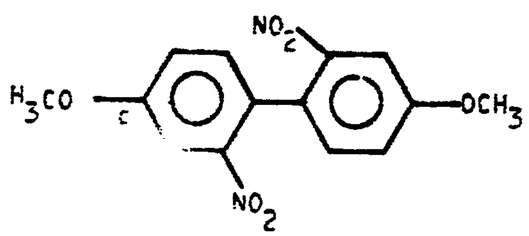 (V)

c) réduire les groupements $NO_2$ du dinitro-2,2' diméthoxy-4,4' biphényle de formule (V) en groupements $NH_2$ pour obtenir le diamino-2,2' diméthoxy-4,4' biphényle de formule :

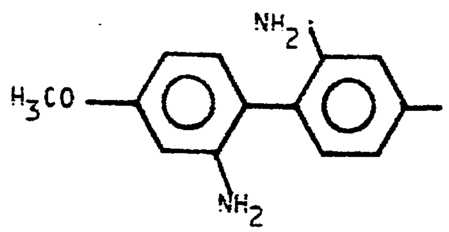 (VI)

d) remplacer les groupements $NH_2$ du diamino-2,2' diméthoxy-4,4' biphényle de formule (VI) par des atomes de fluor pour obtenir le difluoro-2,2' diméthoxy-4,4' biphényle de formule :

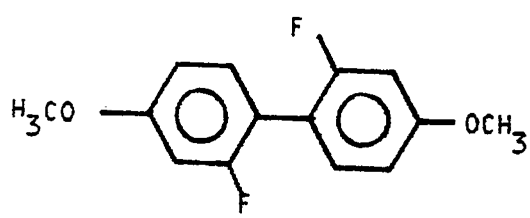 (VII)

e) remplacer les groupes méthoxy du difluoro-2,2' diméthoxy-4,4' biphényle de formule (VII) par des groupes hydroxyle pour obtenir le difluoro-2,2' dihydroxy-4,4' biphényle de formule :

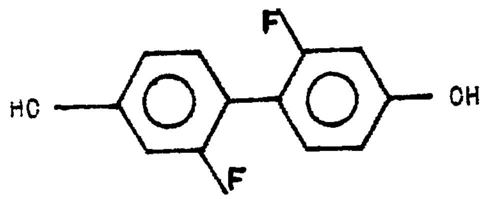
(VIII)

f) faire réagir le difluoro-2,2′ dihydroxy-4,4′ biphényle de formule (VIII) avec un dérivé halogéné de formule $R^2X$ dans laquelle X représente un halogène et $R^2$ représente un radical alkyle de 1 à 12 atomes de carbone, pour obtenir le difluoro-2,2′ alcoxy-4 hydroxy-4′ biphényle de formule (II).

10. Mélange de cristaux liquides comprenant :

- au moins un dérivé de difluoro-2,2′ alcoxy-4 hydroxy-4′ biphényle selon l'une quelconque des revendications 1 à 5, et
- au moins un composé choisi parmi :

a) les bases de Schiff répondant à la formule :

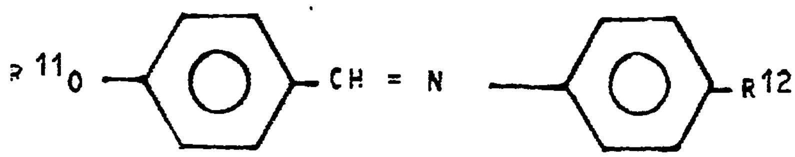
(XIV)

dans laquelle $R^1$ et $R^2$ qui peuvent être identiques ou différents sont des radicaux alkyle de 1 à 7 atomes de carbone,

b) les 1-(alkylcyclohexyl)-2-(alkylfluorobiphénylyl) éthane de formule :

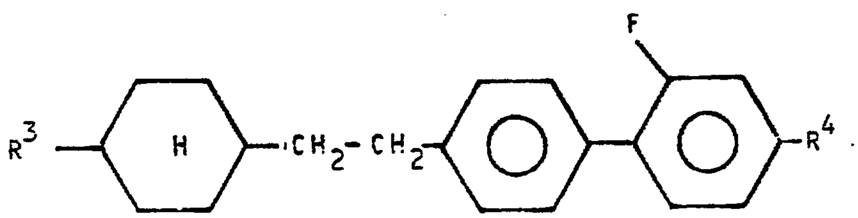
(XV)

dans laquelle $R^3$ et $R^4$ qui peuvent être identiques ou différents, sont des radicaux alkyle de 1 à 7 atomes de carbone, et

c) les alkylbicyclooctane carboxylates d'alkyl fluorophényle de formule :

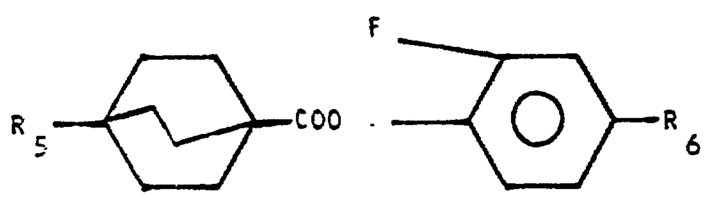
(XVI)

dans laquelle $R_5$ et $R_6$ qui peuvent être identiques ou différents, sont des radicaux alkyle de 1 à 7 atomes de carbone,

d) les cyclohexyl biphényl de formule :

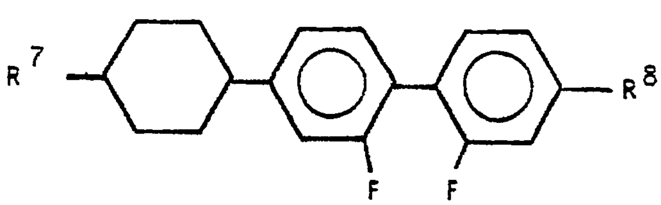
(XVII)

dans laquelle $R_7$ est un radical alkyle de 1 à 12 atomes de carbone et $R_8$ est un radical alkyle ou alkoxy de 1 à 12 atomes de carbone.

11. Mélange de cristaux liquides selon la revendication 10, caractérisé en ce que la teneur du mélange en composé(s) de formule (I) représente au moins 10% en poids du mélange.

12. Dispositif d'affichage à cristal liquide utilisant l'effet de biréfringence contrôlé électriquement, caractérisé en ce que le cristal liquide comprend au moins un dérivé de difluoro-2,2′ alcoxy-4 hydroxy-4′ biphényle selon l'une quelconque des revendications 1 à 5.

## Claims

1. 2,2′-difluoro-4-alkoxy-4′-hydroxydiphenyl derivative according to formula:

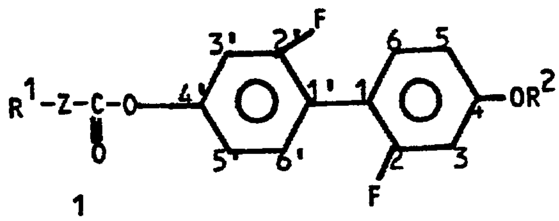 (I)

in which $R^1$ represents a straight or branched alkoxy or alkyl radical with 1 to 12 carbon atoms, Z represents a single bond or a radical chosen from among:

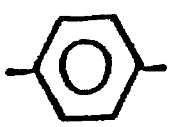 ; 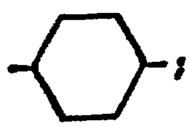 ; 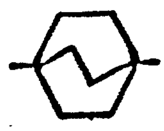 or 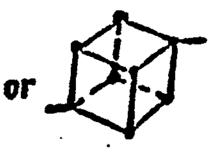

provided that $R^1$ represents an alkyl radical when Z represents a single bond and $R^2$ represents a straight or branched alkyl radical with 1 to 12 carbon atoms.

2. 2,2′-difluoro-4-alkoxy-4′-hydroxydiphenyl derivative according to claim 1, characterized in that it is in accordance with formula:

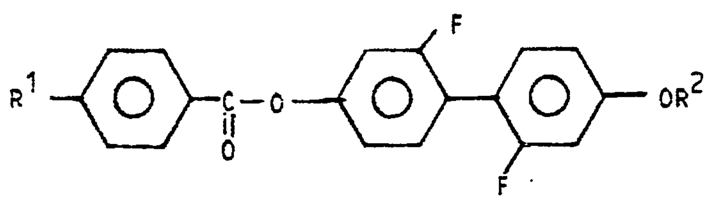 (X)

in which $R^1$ is the butyloxy radical and $R^2$ the ethyl radical, or in which $R^1$ is the decyloxy radical and $R^2$ the 2-methylbutyl radical (S).

3. 2,2′-difluoro-4-alkoxy-4′-hydroxydiphenyl derivative according to claim 1, characterized in that it is in accordance with the formula:

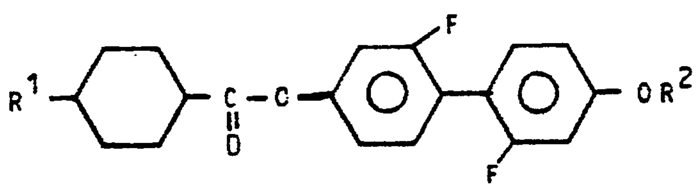 (XI)

in which $R^1$ is an alkyl radical with 3 to 5 carbon atoms and $R^2$ an alkyl radical with 2 to 5 carbon atoms.

4. 2,2′-difluoro-4-alkoxy-4′-hydroxydiphenyl derivative according to claim 1, characterized in that it complies with the formula:

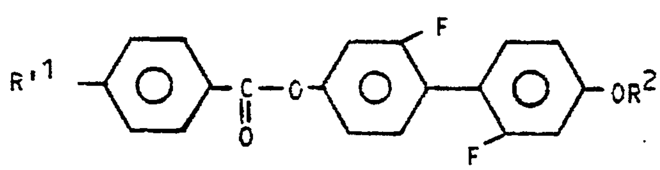 (XII)

in which $R^1$ is an alkyl radical with 6 to 8 carbon atoms and $R^2$ an alkyl radical with 2 to 5 carbon atoms.

5. 2,2′-difluoro-4-alkoxy-4′-hydroxydiphenyl derivative according to claim 1, characterized in that it complies with the formula:

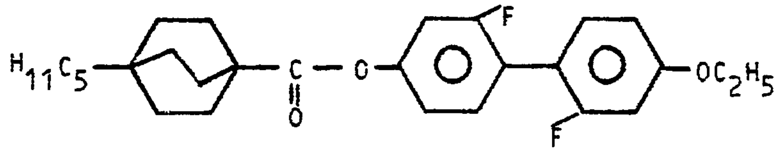

(XIII)

6. Process for the preparation of a 2,2′-difluoro-4-alkoxy-4′-hydroxydiphenyl derivative according to formula:

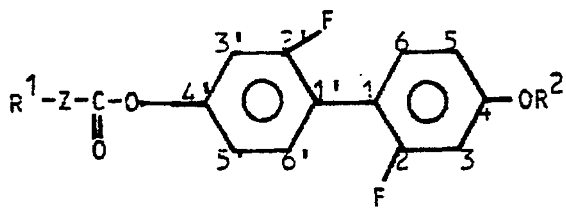

(I)

in which $R^1$ represents a straight or branched alkoxy or alkyl radical with 1 to 12 carbon atoms and Z represents a single bond or a radical chosen from among:

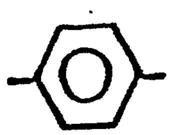 ; 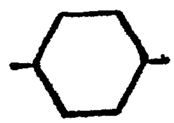 ; 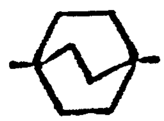 or 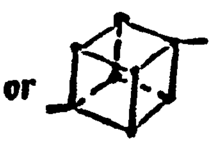

provided that $R^1$ represents an alkyl radical when Z represents a single bond and $R^2$ represents a straight or branched alkyl radical with 1 to 12 carbon atoms, characterized in that an acid chloride of formula $R^1$–Z–COCl, in which $R^1$ and Z have the meanings given hereinbefore, is reacted with a compound of formula:

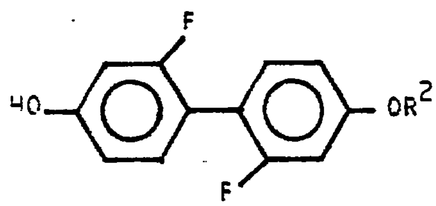

(II)

in which $R^2$ has the meaning given hereinbefore.

7. 2,2′-difluoro-4-alkoxy-4′-hydroxydiphenyl according to formula:

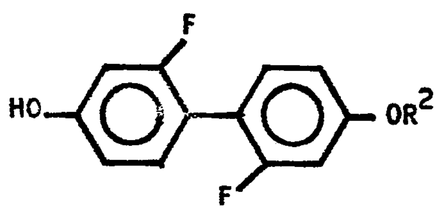

(II)

in which $R^2$ represents a straight or branched alkyl radical with 1 to 12 carbon atoms.

8. 2,2′-difluoro-4-alkoxy-4′-diphenyl according to claim 7, characterized in that $R^2$ is chosen from among the ethyl propyl butyl n-pentyl and 2-methyl butyl radicals.

9. Process for the preparation of a 2,2′-difluoro-4-alkoxy-4′-hydroxy diphenyl according to formula:

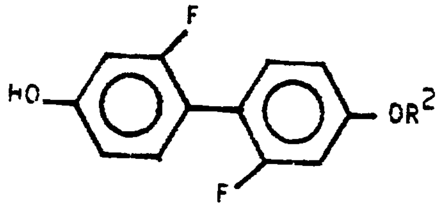

(II)

in which $R^2$ represents a straight or branched alkyl radical with 1 to 12 carbon atoms, characterized in that it comprises the following stages:

a) converting the 3-nitro-4-aminoanisole of formula:

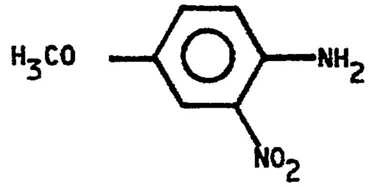

(III)

into 3-nitro-4-iodoanisole of formula:

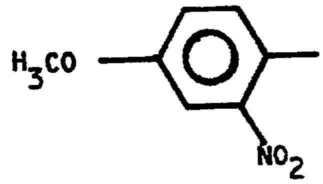

(IV)

by reaction with nitrous acid or an aqueoùs solution of alkali metal nitrite and alkali metal iodide,
b) condensing two molecules of the 3-nitro-4-iodoanisole of formula (IV) to obtain 2,2'-dinitro-4,4'-dimethoxydiphenyl of formula:

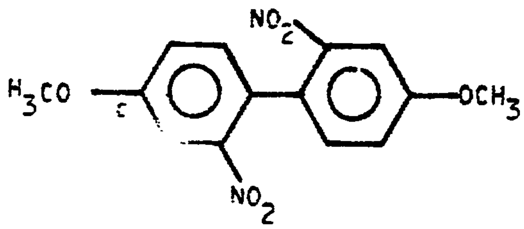

(V)

c) reducing the $NO_2$ groups of 2,2'-dinitro-4,4'-dimethoxy diphenyl of formula (V) into $NH_2$ groups to obtain the 2,2'-diamino-4,4'-dimethoxy diphenyl of formula:

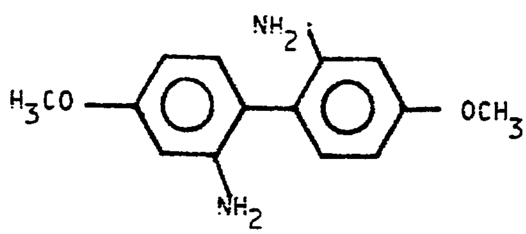

(VI)

d) replacing the $NH_2$ groups of the 2,2'-diamino-4,4'-dimethoxy diphenyl of formula (VI) by fluorine atoms to obtain the 2,2'-difluoro-4,4'-dimethoxydiphenyl of formula:

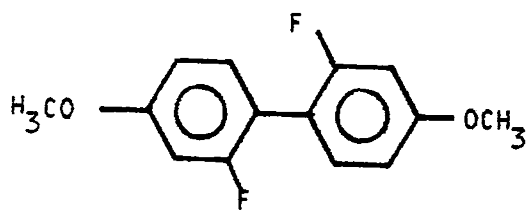

(VII)

e) replacing the methoxy groups of the 2,2'-difluoro-4,4'-dimethoxy diphenyl of formula (VII) by hydroxyl groups to obtain the 2,2'-difluoro-4,4'-dihydroxydiphenyl of formula:

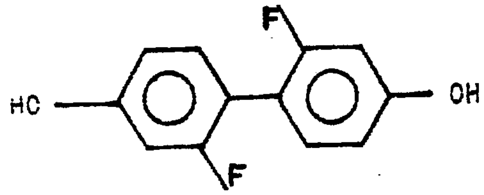

(VIII)

f) reacting the 2,2′-difluoro-4,4′-dihydroxydiphenyl of formula (VIII) with a halogen derivative of formula $R^2X$, in which X represents a halogen and $R^2$ represents an alkyl radical with 1 to 12 carbon atoms, to obtain the 2,2′-difluoro-4-alkoxy-4′-hydroxydiphenyl of formula (II).

10. Mixture of liquid crystals comprising at least one 2,2′-difluoro-4-alkoxy-4′-diphenyl derivative according to one of the claims 1 to 5 and at least one compound chosen from among:

a) the Schiff bases complying with formula:

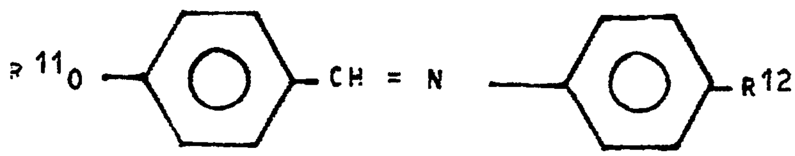

(XIV)

in which $R^1$ and $R^2$, which can be the same or different, are alkyl radicals with 1 to 7 carbon atoms,

b) 1-(alkylcyclohexyl)-2-(alkylfluorodiphenylyl)-ethane of formula:

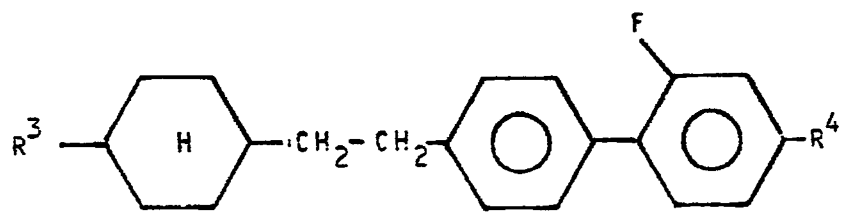

(XV)

in which $R^3$ and $R^4$, which can be the same or different, are alkyl radicals with 1 to 7 carbon atoms and

c) the alkylfluorophenyl-alkylbicyclooctane carboxylates of formula:

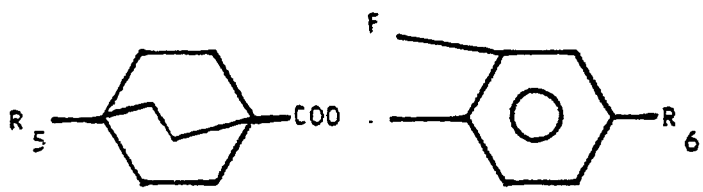

(XVI)

in which $R_5$ and $R_6$, which can be the same or different, are alkyl radicals with 1 to 7 carbon atoms,

d) the cyclohexyl diphenyl of formula:

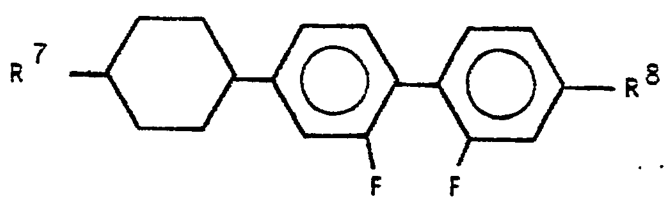

(XVII)

in which $R_7$ is an alkyl radical with 1 to 12 carbon atoms and $R_8$ an alkyl or alkoxy radical with 1 to 12 carbon atoms.

11. Mixture of liquid crystals according to claim 10, characterized in that the content of the mixture in compounds of formula (I) represents at least 10% by weight of the mixture.

12. Liquid crystal display device using the electrically controlled birefringence effect, characterized in that the liquid crystal comprises at least one 2,2′-difluoro-4-alkoxy-4′-hydroxydiphenyl derivative according to any one of the claims 1 to 5.

**Patentansprüche**

1. 2,2'-Difluoro-4-alkoxy-4'-hydroxy-biphenylderivat der Formel

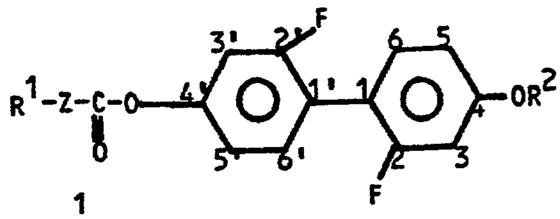 (I)

worin bedeuten:
$R^1$ einen linearen oder verzweigten Alkyl- oder Alkoxyrest mit 1 bis 12 Kohlenstoffatomen,
Z eine einfache Bindung oder einen Rest, ausgewählt aus

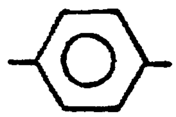 ; 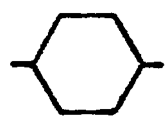 ; 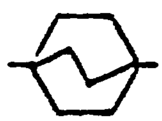 oder 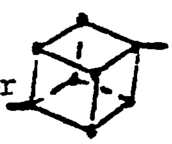

mit der Maßgabe, daß $R^1$ einen Alkylrest darstellt, wenn Z für eine einfache Bindung steht, und
$R^2$ einen linearen oder verzweigten Alkylrest mit 1 bis 12 Kohlenstoffatomen.

2. 2,2'-Difluoro-4-alkoxy-4'-hydroxybiphenylderivat nach Anspruch 1, dadurch gekennzeichnet, daß es der Formel entspricht:

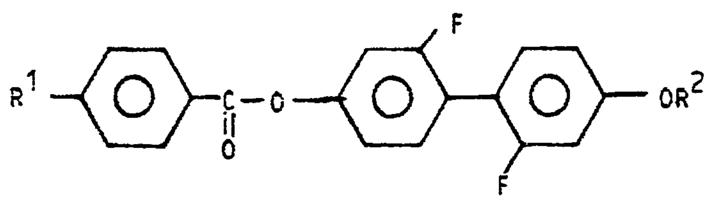 (X)

worin bedeuten:
$R^1$ den Butyloxyrest und
$R^2$ den Ethylrest oder
$R^1$ den Decyloxyrest und
$R^2$ den (S)-2-Methylbutylrest.

3. 2,2'-Difluoro-4-alkoxy-4'-hydroxy-biphenylderivat nach Anspruch 1, dadurch gekennzeichnet, daß es der Formel entspricht:

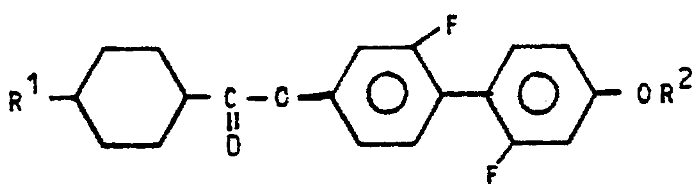 (XI)

worin bedeuten:
$R^1$ einen Alkylrest mit 3 bis 5 Kohlenstoffatomen und
$R^2$ einen Alkylrest mit 2 bis 5 Kohlenstoffatomen.

4. 2,2'-Difluoro-4-alkoxy-4'-hydroxy-biphenylderivat nach Anspruch 1, dadurch gekennzeichnet, daß es der Formel entspricht:

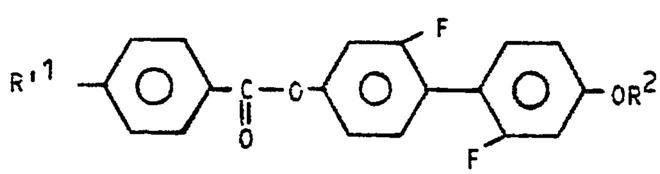 (XII)

worin bedeuten:
$R^1$ einen Alkylrest mit 6 bis 8 Kohlenstoffatomen und
$R^2$ einen Alkylrest mit 2 bis 5 Kohlenstoffatomen.

5. 2,2'-Difluoro-4-alkoxy-4'-hydroxy-biphenylderivat nach Anspruch 1, dadurch gekennzeichnet, daß es der Formel entspricht

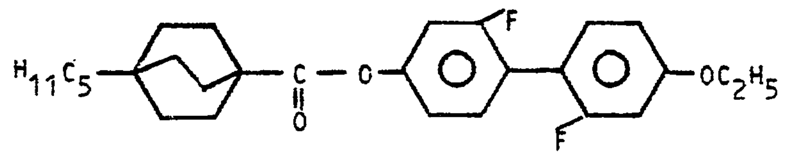 (XIII)

6. Verfahren zur Herstellung eines 2,2'-Difluoro-4-alkoxy-4'-hydroxy-biphenylderivats der Formel

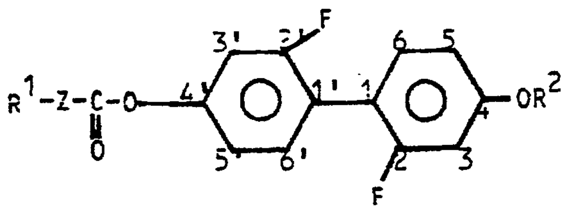 (I)

worin bedeuten:
$R^1$ einen linearen oder verzweigten Alkyl- oder Alkoxyrest mit 1 bis 12 Kohlenstoffatomen,
Z eine einfache Bindung oder eine Rest, ausgewählt aus

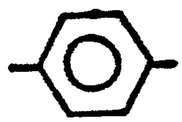 ; 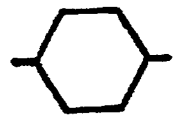 ; 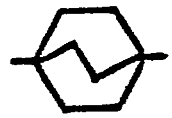 oder 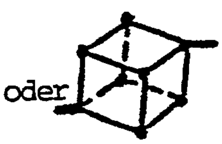

mit der Maßgabe, daß $R^1$ einen Alkylrest darstellt, wenn Z für eine einfache Bindung steht, und
$R^2$ einen linearen oder verzweigten Alkylrest mit 1 bis 12 Kohlenstoffatomen,
dadurch gekennzeichnet, daß man ein Säurechlorid der Formel
$R^1$–Z–COCl
worin $R^1$ und Z die oben angegebenen Bedeutungen haben,
reagieren läßt mit einer Verbindung der Formel

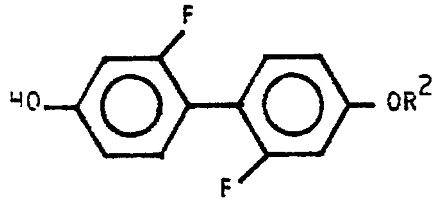 (II)

worin $R^2$ die oben angegebene Bedeutung hat.

7. 2,2'-Difluoro-4-alkoxy-4'-hydroxy-biphenyl der Formel

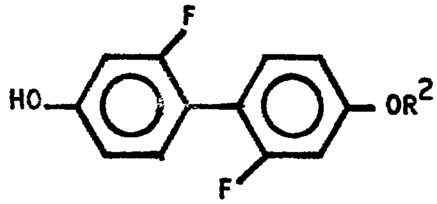 (II)

worin $R^2$ einen linearen oder verzweigten Alkylrest mit 1 bis 12 Kohlenstoffatomen bedeutet.

8. 2,2'-Difluoro-4-alkoxy-4'-hydroxy-biphenyl nach Anspruch 7, dadurch gekennzeichnet, daß $R^2$ ausgewählt wird aus den Ethyl-, Propyl-, Butyl-, n-Pentyl- und 2-Methylbutyl-Resten.

9. Verfahren zur Herstellung eines 2,2'-Difluoro-4-alkoxy-4'-hydroxy-biphenyls der Formel

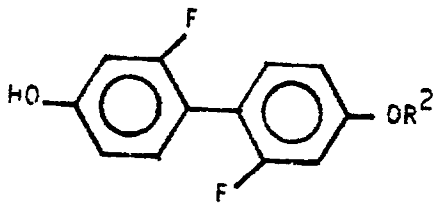

(II)

worin $R^2$ einen linearen oder verzweigten Alkylrest mit 1 bis 12 Kohlenstoffatomen bedeutet, dadurch gekennzeichnet, daß es die folgenden Stufen umfaßt:

(a) Umwandlung des 3-Nitro-4-amino-anisols der Formel

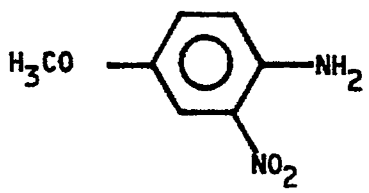

(III)

in das 3-Nitro-4-jodo-anisol der Formel

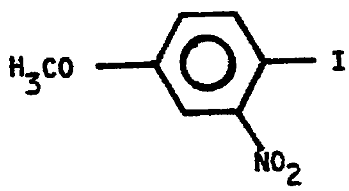

(IV)

durch Umsetzung mit Salpetriger Säure oder einer wäßrigen Lösung eines Alkalimetallnitrits und eines Alkalimetalljodids,

(b) Kondensieren von zwei Molekülen des 3-Nitro-4-jodoanisols der Formel (IV) zur Herstellung des 2,2′-Dinitro-4-4′-dimethoxy-biphenyls der Formel

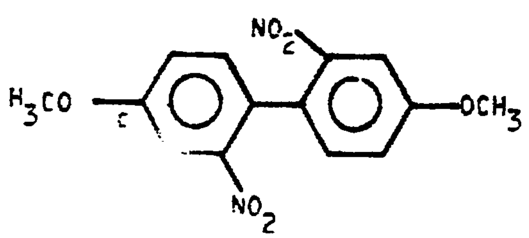

(V)

(c) Reduzieren der $NO_2$-Gruppen des 2,2′-Dinitro-4,4′-dimethoxy-biphenyls der Formel (V) zu $NH_2$-Gruppen zur Herstellung des 2,2′-Diamino-4,4′-dimethoxy-biphenyls der Formel

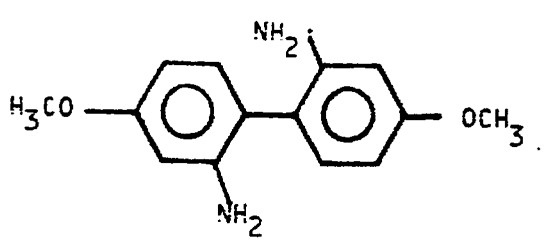

(VI)

(d) Ersatz der $NH_2$-Gruppen des 2,2′-Diamino-4,4′-dimethoxy-biphenyls der Formel (VI) durch Fluoratome zur Herstellung des 2,2′-Difluoro-4,4′-dimethoxy-biphenyls der Formel

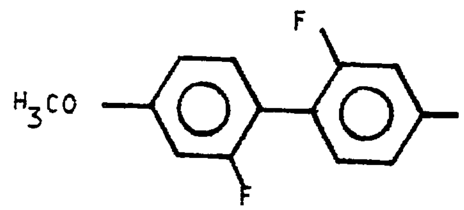

(VII)

e) Ersatz der Methoxygruppen des 2,2'-Difluoro-4,4'-dimethoxy-biphenyls der Formel (VII) durch Hydroxylgruppen zur Herstellung des 2,2'-Difluoro-4,4'-dihydroxy-biphenyls der Formel

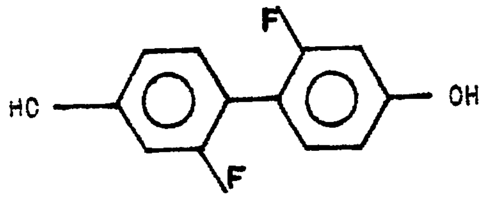

(VIII)

(f) Reagierenlassen des 2,2'-Difluoro-4,4'-dihydroxy-biphenyls der Formel (VIII) mit einem Halogenderivat der Formel $R^2X$, worin X für ein Halogen steht und $R^2$ einen Alkylrest mit 1 bis 12 Kohlenstoffatomen darstellt, zur Herstellung des 2,2'-Difluoro-4-alkoxy-4'-hydroxy-biphenyls der Formel (II).

10. Flüssigkristallgemisch, das umfaßt:

– mindestens ein 2,2'-Difluoro-4-alkoxy-4'-hydroxy-biphenylderivat nach einem der Ansprüche 1 bis 5 und

– mindestens eine Verbindung, die ausgewählt wird aus:

(a) Schiff'schen Basen der Formel

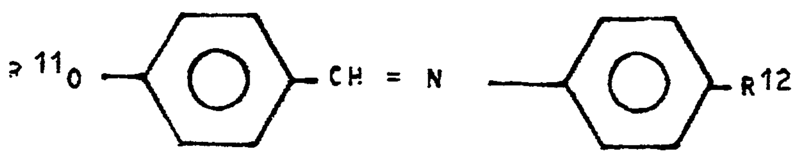

(XIV)

worin $R^{11}$ und $R^{12}$, die gleich oder verschieden sein können, Alkylreste mit 1 bis 7 Kohlenstoffatomen darstellen,

(b) 1-(Alkylcyclohexyl)-2-(alkylfluorobiphenylyl)ethanen der Formel

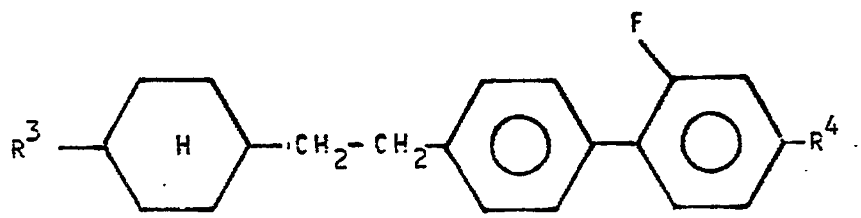

(XV)

worin $R^3$ und $R^4$, die gleich oder verschieden sein können, Alkylreste mit 1 bis 7 Kohlenstoffatomen darstellen, und

(c) Alkylbicyclooctancarboxylaten von Alkylfluorophenyl der Formel

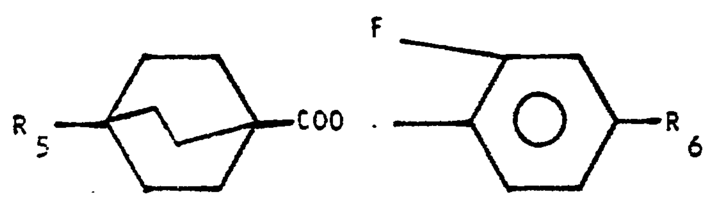

(XVI)

worin $R^5$ und $R^6$, die gleich oder verschieden sein können, Alkylreste mit 1 bis 7 Kohlenstoffatomen darstellen, und

(d) Cyclohexylbiphenylen der Formel

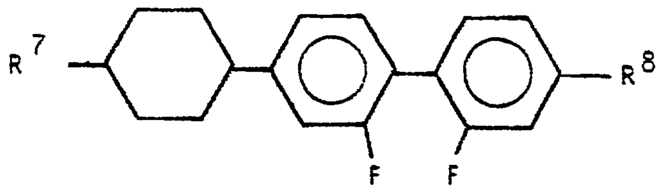

(XVII)

worin $R^7$ einen Alkylrest mit 1 bis 12 Kohlenstoffatomen und $R^8$ einen Alkyl- oder Alkoxyrest mit 1 bis 12 Kohlenstoffatomen darstellen.

11. Flüssigkristallgemisch nach Anspruch 10, dadurch gekennzeichnet, daß der Gehalt des Gemisches an Verbindung(en) der Formel (I) mindestens 10 Gew.-% des Gemisches ausmacht.

12. Flüssigkristall-Anzeigeelement, in dem der elektrisch kontrollierte Doppelbrechungseffekt ausgenutzt wird, dadurch gekennzeichnet, daß der Flüssigkristall umfaßt mindestens ein 2,2'-Difluoro-4-alkoxy-4'-hydroxy-biphenylderivat nach einem der Ansprüche 1 bis 5.

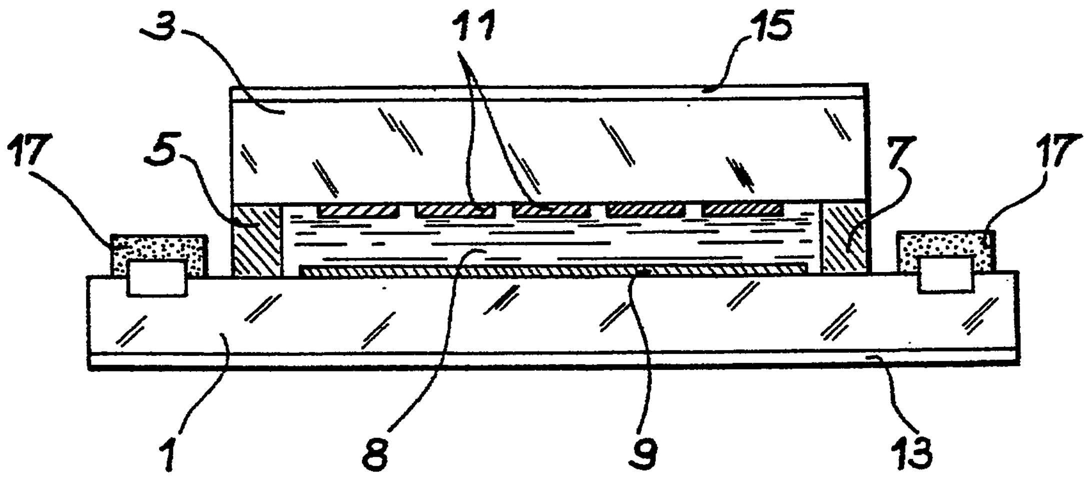
3
11
15
17
5
7
17
1
8
9
13